# EUROPÄISCHE PATENTANMELDUNG

(11) **EP 1 477 556 A1**
(43) Veröffentlichungstag der Anmeldung: **17.11.2004**
(21) Anmeldenummer: 03011019.1
(22) Anmeldetag: 16.05.2003
(51) Int. Cl.: C12N 5/06, C12N 15/86

(54) **Verfahren zur Selektion von kardiomyogenen Zellen oder Herzmuskelzellen aus gemischten Zellpopulationen**

(71) Anmelder: Heart BioSystems GmbH, 69120 Heidelberg (DE)
(72) Erfinder: Küpper, Jan-Heiner, 74909 Meckesheim (DE); Kuhn, Anne, 69221 Heidelberg (DE)
(74) Vertreter: Isenbruck, Günter, Dr.

(57) **Zusammenfassung**

Die vorliegende Erfindung betrifft ein Verfahren zur in vitro Selektion von kardiomyogenen Zellen oder Herzmuskelzellen aus einer Mischung mit anderen Zelltypen, enthaltend die Schritte (a) Einbringen mindestens eines Selektionskonstruktes, das auf einem nicht in das zelluläre Genom integrierenden Vektor enthalten ist, in die zu selektierenden Zellen, wobei auf dem Selektionskonstrukt mindestens ein Selektionsmarker kodiert ist, der spezifisch in kardiomyogenen Zellen oder Herzmuskelzellen exprimiert wird, und (b) Kultivierung der Zellen unter Zugabe mindestens eines cytotoxischen oder cytostatischen Selektionsmittels bis zum Erhalt einer reinen Zellpopulation kardiomyogener Zellen oder Herzmuskelzellen. Ferner betrifft die Erfindung ein Verfahren zur Herstellung reiner Kulturen kardiomyogener Zellen oder Herzmuskelzellen, die Verwendung reiner Kulturen kardiomyogener Zellen oder Herzmuskelzellen, sowie Zellkultur-Banken mit gewebetypisch charakterisierten Kulturen kardiomyogener Zellen und Herzmuskelzellen.

## Beschreibung

Die Erfindung betrifft ein Verfahren zur Selektion von kardiomyogenen Zellen oder Herzmuskelzellen, ein Verfahren zur Herstellung reiner Kulturen kardiomyogener Zellen oder Herzmuskelzellen, die Verwendung reiner Kulturen kardiomyogener Zellen oder Herzmuskelzellen, sowie Zellkultur-Banken mit gewebetypisch charakterisierten Kulturen kardiomyogener Zellen und Herzmuskelzellen.

Herz- und Kreislauferkrankungen sind die führende Todesursache in allen Industrieländern. Eine der häufigsten Erkrankungen des Herzkreislaufsystems, die Atherosklerose, führt zu Ablagerungen in den Blutgefäßen und damit zu starken Verengungen (Stenosen) der Gefäße. Bilden sich diese Ablagerungen in den Herzkranzgefäßen, die den Herzmuskel versorgen, werden die entsprechenden Bereiche des Herzmuskels nur unzureichend mit Sauerstoff versorgt und die Herzmuskelzellen sterben in diesen Bereichen ab. Diese irreversible Schädigung des Herzmuskels führt zum Herzinfarkt (Absterben des Herzmuskels wegen Sauerstoffmangels), an dem etwa ein Drittel aller Patienten versterben, während die überlebenden Patienten häufig mit Komplikationen wie Herzrhythmusstörungen zu kämpfen haben. Auch bei anderen Herzerkrankungen tritt häufig ein irreversibler Verlust von Herzmuskelzellen ein.

Da bislang keine Therapie zur Regeneration von Herzmuskelzellen im lebenden Körper (in vivo) existiert, wird die Transplantation von Zellen bzw. Gewebe- oder Organteilen (Tissue Engineering) als derzeit einzige durchführbare Möglichkeit zur Behandlung solcher Patienten angesehen. Allerdings stellt die Gewinnung derartiger Zellen ein großes Problem dar, nicht zuletzt aufgrund der Tatsache, dass Herzmuskelzellen des erwachsenen Organismus nicht mehr zur Teilung und Vermehrung in der Lage sind. In den vergangenen Jahren wurden daher verschiedene Ansätze verfolgt, solche Zellen zu gewinnen und zu kultivieren. Vor allem die Erforschung von embryonalen Stammzellen wurde in den letzten Jahren stark vorangetrieben, da diese Zellen totipotent sind, d.h. sie sind prinzipiell in der Lage, sich in alle Zelltypen zu differenzieren und können somit zur Regeneration der unterschiedlichsten Gewebe herangezogen werden.

Ein anderer Ansatz verfolgt die Erforschung adulter Stammzellen, welche nur pluripotent sind, d.h. sich nur in eine begrenzte Zahl von Zelltypen differenzieren können. Da es aber verschiedene Populationen von adulten Stammzellen mit jeweils unterschiedlichem Entwicklungspotential gibt, können wahrscheinlich alle Zelltypen des Körpers auch aus adulten Stammzellen gebildet werden. Trotzdem ist der Umgang mit adulten Stammzellen schwieriger.

Besonders interessant sind derzeit Verfahren, bei denen Herzmuskelzellen oder Vorläufer von Herzmuskelzellen (kardiomyogene Zellen) in vitro gewonnen werden, um sie dann in das Herz zu transplantieren. Dabei erscheinen diejenigen Ansätze am attraktivsten, bei denen immunverträgliches Zellmaterial verwendet wird, welches also nicht vom Empfänger abgestoßen wird. Nach Möglichkeit sollten die Zellen vom Patienten selber stammen. Derzeit gibt es im Wesentlichen zwei unterschiedliche Ansätze:
1. Verwendung embryonaler Stammzellen (ES-Zellen) als Ausgangsmaterial für Gewebe.
   ES-Zellen sind außerhalb des Körpers prinzipiell unbegrenzt teilungsfähig und es besteht die Möglichkeit, organähnliche Strukturen (Organoide) aus embryonalen Stammzellen zu differenzieren. Diese Organoide können auch spontan differenzierte Herzmuskelzellen enthalten.
2. Verwendung mesenchymaler Stammzellen aus Knochenmark, die außerhalb des Körpers in Herzmuskelzellen umgewandelt werden.
   Mesenchymale Stammzellen stellen eine Population von adulten Knochenmarkstammzellen dar, die sich in Herzmuskelzellen differenzieren können.

Großer Nachteil der Verwendung von ES-Zellen ist neben ethischen Bedenken die Notwendigkeit des Kerntransfers. Nur nach Transfer eines Patienten-Zellkerns in die befruchtete Eizelle haben die sich daraus entwickelnden Zellen die immunologischen Eigenschaften des jeweiligen Patienten / Zellkemspenders. Leider ist der Kerntransfer insgesamt technologisch äußerst aufwendig und führt häufig zu genetischen Defekten. Ferner sind ES-Zellen als totipotente Zellen fähig, sogenannten Teratokarzinomen ähnliche Tumore zu bilden. Die ES-Zellen müssten daher vor der Verwendung vollständig ausdifferenziert werden, was schwer zu kontrollieren ist. Umgekehrt ist es unklar, ob vollständig ausdifferenzierte Zellen über eine hinreichende Fähigkeit zur Integration in den bereits existierenden Gewebeverband eines Herzens verfügen. Weniger differenzierte Vorläuferzellen von Herzmuskelzellen, kardiomyogene Zellen, dürften über eine bessere Fähigkeit verfügen, sich in den existierenden Gewebeverband einzufügen und in vivo auszudifferenzieren.

Daher stellt eine Differenzierung adulter Stammzellen in vitro den derzeit vielversprechendsten Weg zur Bereitstellung hinreichend großer Zahlen von kardiomyogenen Zellen oder Herzmuskelzellen dar. Adulte Stammzellen, insbesondere mesenchymale Knochenmarkstammzellen, lassen sich z.B. durch Gabe der Substanz 5'-Azazytidin oder durch Kokultivierung mit Herzmuskelzellen in kardiomyogene Zellen, d.h. Vorläufer von Herzmuskelzellen, differenzieren.

Alle bekannten Differenzierungsverfahren sind aber ineffizient, d.h. es entstehen auch andere Zelltypen als kardiomyogene Zellen oder Herzmuskelzellen. Dies betrifft auch die Differenzierung aus anderen Ausgangszellen als mesenchymalen Knochenmarkstammzellen. Daher besteht das Problem, kardiomyogene Zellen und Herzmuskelzellen von den anderen Zellen zu isolieren. Bei Verwendung der Kokultivierungsmethode müssen auch die zur Differenzierungsauslösung benutzten Zellen vollständig von den differenzierten Zellen entfernt werden.

Ohne ein Selektionsverfahren ist es somit nicht möglich, die gewonnenen kardiomyogenen Zellen und Herzmuskelzellen in ausreichender Zahl und Reinheit anzureichern. Die folgenden drei Selektionsverfahren für Herzmuskelzellen sind bekannt. Alle Verfahren beinhalten das Einbringen von DNA-Konstrukten, die stabil in das Genom integrieren.

Ein Selektionsverfahren für Herzmuskelzellen wird von Müller et al. (FASEB J., 14: 2540-2548, 2000) beschrieben. Bei dem Verfahren wird ein DNA-Konstrukt in ES-Zellen eingebracht, bei welchem GFP (grün fluoreszierendes Protein) unter Kontrolle des Myosin Light Chain-2v (MLC-2v) Promotors der Ratte steht. Bei Bildung sogenannter Embryoid Bodies, d.h. aus den ES-Zellen aggregierenden Zellverbänden, differenzieren sich spontan kontrahierende Zellen.

In dem Dokument wird gezeigt, dass innerhalb der Embryoid Bodies diejenigen Zellen, die GFP exprimieren, Charakteristika von Herzmuskelzellen aufweisen. Die GFP exprimierenden Zellen konnten nach enzymatischer Dissoziation der Embryoid Bodies mit einem automatisierten Sortierverfahren, FACS (fluorescence activated cell sorting), von anderen Zellen getrennt, also selektioniert werden.

Franz et al. (US-Patent 5,928,943) beschreiben die Verwendung zweier DNA-Konstrukte. Das erste Konstrukt erlaubt die Selektion auf transfizierte ES-Zellen nach der Elektroporation. Das zweite Konstrukt enthält ein Fusionsgen aus den Genen für β-Galaktosidase und dem Neomycin-Resistenzgen unter Kontrolle von 2,1 kb des MLC-2v Promotors. Diejenigen ES-Zellklone, die beide Konstrukte enthalten, werden in dem Verfahren weiter vermehrt und wiederum zu Embryoid Bodies entwickelt, wobei innerhalb der Embryoid Bodies spontan auch Herzmuskelzellen differenzieren. Innerhalb der Embryoid Bodies ist der MLC-2v Promotor nur in den Herzmuskelzellen aktiv, so dass nur in diesen Zellen das Neomycin-Resistenzgen exprimiert wird (aktiv ist) und eine Resistenz gegen das Antibiotikum G418 (Geneticin) verleiht. Bei Gabe von G418 überleben also nur die Herzmuskelzellen. Die auf diese Weise aus Embryoid Bodies selektierten Herzmuskelzellen repräsentieren aber nicht die frühesten kardiomyogenen Entwicklungsstadien, da es sich schon um kontraktile Zellen handelt.

In WO 01/071006 wird ein Verfahren beschrieben, bei dem die Expression eines Zelloberflächenrezeptors unter Kontrolle des in Herzmuskelzellen aktiven MLC-2v Promotors steht. Zur Selektion werden an magnetische Microbeads gekoppelte Antikörper verwendet, die spezifisch an den Zelloberflächenrezeptor binden. Somit bindet der Antikörper an Zellen, die den Zelloberflächenrezeptor exprimieren, also mutmaßliche Herzmuskelzellen. Die so markierten Zellen können in einem Magnetfeld isoliert werden. Dafür müssen die Zellen in einem speziellen nicht-enzymatischen Verfahren vom Boden der Zellkulturschale gelöst werden.

Die beschriebenen Verfahren beziehen sich fast ausschließlich auf Herzmuskelzellen, die aus ES-Zellen gewonnen wurden. Die spontane Differenzierung ist wenig reproduzierbar und die Zahl der erzeugten Zellen gering. Bei aus ES-Zellen differenzierten heterologen Herzmuskelzellen, bzw. bei heterologen Zellen allgemein, müssen zusätzliche Vorkehrungen getroffen werden, um eine Abstoßung durch das Immunsystem des Empfängers zu vermeiden (siehe z.B. Franz, WO 01/071006). Weitere Nachteile der Verwendung von ES-Zellen wurden oben bereits erwähnt.

Sämtlichen bekannten Verfahren ist gemeinsam, dass die DNA-Konstrukte dauerhaft (stabil) in das Genom der selektierten Zellen integrieren und die Zellen genetisch verändert werden. Dauerhafte Integration hat den Vorteil, dass die Zelllinien dauerhaft selektiert werden können. Es gibt aber auch schwerwiegende Nachteile:
1. Die integrierten Gene werden zeitlich unbegrenzt exprimiert, was zu unvorhersehbaren Auswirkungen auf den Empfänger führen kann. Zum einen können die exprimierten Proteine (z.B. GFP) die Zellen selber schädigen und zum Absterben der Zellen führen. Dies ist gerade bei nicht regenerationsfähigen Zellen wie Herzmuskelzellen nicht tolerabel. Ferner können die dauerhaft exprimierten Proteine zu einer Immunreaktion des Empfängers führen, unter Umständen bis hin zum anaphylaktischen Schock mit Todesfolge.
2. Die Integration ins Genom erfolgt üblicherweise zufällig, so dass es auch zu Integrationen in körpereigene Gene kommen kann. Solche körpereigenen Gene wären dann mutiert (sog. Insertionsmutagenese), was zu Krebsentstehung führen kann, insbesondere wenn das Gen an der Kontrolle des Zellzyklus beteiligt ist. Entsprechend transformierte Zellen sind schneller teilungsfähig als andere Zellen. Sie würden daher bevorzugt vermehrt und in den Empfänger reimplantiert.
3. Die Expression des Markergenes wird von der Umgebung der Integrationsstelle im Chromosom beeinflusst. Einige Zellen werden daher das Markergen exprimieren, obwohl sie keine Charakteristika von Herzmuskelzellen haben. Dies kann dazu führen, dass Zellen transplantiert werden, die für das Empfängerherz schädlich sind. Andererseits werden einige differenzierte Herzmuskelzellen das Transgen nicht exprimieren, da das Transgen an einer Stelle im Chromosom integriert ist, an der es nicht exprimiert wird.

Den bisherigen Verfahren ist ferner gemeinsam, dass sie für Zellen tierischer Herkunft (Maus ES-Zellen) durchgeführt wurden. Dabei wurden nur geringe Mengen an Zellen erzeugt, die für eine klinische Anwendung nicht ausreichen würden.

Ziel der vorliegenden Erfindung ist es, ein Verfahren zur Selektion kardiomyogener Zellen oder Herzmuskelzellen zur Verfügung zu stellen, das nicht die Nachteile der Verfahren nach dem Stand der Technik aufweist. Insbesondere ist es Ziel der vorliegenden Erfindung ein klinisch durchführbares, sicheres und ethisch vertretbares Verfahren zur Verfügung zu stellen. Ganz besonders ist es Ziel der vorliegenden Erfindung, ein Verfahren zur Verfügung zu stellen, bei dem die Nachteile dauerhafter Integration vermieden werden, und welches immunverträglich und schnell durchführbar ist.

Bei dem erfindungsgemäßen Verfahren wird ein Selektionsmarkergen nur transient in die Zellen eingebracht, so dass die Zellen durch das Selektionsmarkergen nicht dauerhaft genetisch verändert werden und eine Insertionsmutagenese vermieden wird. Trotz des nur transienten Verbleibs des Selektionsmarkergens in den Zellen ist eine vollständige Selektion möglich.

Das erfindungsgemäße Verfahren zur in vitro Selektion von kardiomyogenen Zellen oder Herzmuskelzellen aus einer Mischung mit anderen Zelltypen enthält die Abfolge der folgenden Schritte:
a) Einbringen mindestens eines Selektionskonstruktes, das auf einem nicht in das zelluläre Genom integrierenden Vektor enthalten ist, in die zu selektierenden Zellen, wobei auf dem Selektionskonstrukt mindestens ein Selektionsmarker kodiert ist, der spezifisch in kardiomyogenen Zellen oder Herzmuskelzellen exprimiert wird,
b) Kultivierung der Zellen unter Zugabe mindestens eines cytotoxischen oder cytostatischen Selektionsmittels bis zum Erhalt einer reinen Zellpopulation kardiomyogener Zellen oder Herzmuskelzellen.

Mit dem vorliegenden Verfahren können reine Zellpopulationen bzw. Kulturen von kardiomyogenen Zellen oder Herzmuskelzellen gewonnen werden. Insbesondere können reine Zellpopulationen bzw. Kulturen kardiomyogener Zellen gewonnen werden. Insbesondere können die reinen Zellpopulationen bzw. Kulturen aus menschlichen Zellen erzeugt werden. Unter "rein" werden Zellpopulationen bzw. Kulturen verstanden, die weniger als 10 %, insbesondere weniger als 1 %, im Speziellen weniger als 0,1 % Zellen anderer Zelltypen enthalten.

Eine "reine Zellpopulation" im Sinne der Erfindung besteht aus Zellen, die den Selektionsmarker oder den nicht integrierenden Vektor mit dem Selektionskonstrukt enthalten. Gegenstand der Erfindung ist auch eine reine Zellpopulation kardiomyogener Zellen oder Herzmuskelzellen, vorzugsweise eine Zellpopulation, die den Vektor mit dem Selektionskonstrukt enthält.

Eine "reine Kultur" im Sinne der Erfindung besteht dagegen aus Zellen, die das Selekticnskonstrukt nicht mehr enthalten. Gegenstand der Erfindung ist auch eine reine Kultur kardiomyogener Zellen oder Herzmuskelzellen.

Das Verfahren kann auf Zellen aus Tieren und Menschen, bevorzugt Menschen, angewendet werden. Bevorzugte Tiere sind Säugetiere, besonders bevorzugt sind Nagetiere und Primaten.

Die Selektion der kardiomyogenen Zellen und Herzmuskelzellen erfolgt durch Einbringen eines Nukleinsäure-Konstruktes, bevorzugt eines DNA-Konstruktes, (dem Selektionskonstrukt), welches ein Gen für einen Selektionsmarker (z.B. ein Antibiotika-Resistenzgen) unter der Kontrolle einer spezifisch in kardiomyogenen Zellen und Herzmuskelzellen aktiven Expressionssequenz (z.B. MLC-2v Promotor) beinhaltet. Das Selektionskonstrukt bewirkt, dass nur diejenigen Zellen, welche sich in Herzmuskelzellen umgewandelt haben, den Selektionsmarker produzieren und nach Gabe des entsprechenden Selektionsmittels überleben.

Das Selektionskonstrukt wird durch nicht in das zelluläre Genom integrierende Nukleinsäure-Vektoren in die zu differenzierenden Zellen eingebracht. Unter "nicht integrierend" sind im Sinne der Erfindung Vektoren zu verstehen, die bei Verwendung im Rahmen des vorliegenden Verfahrens in das Genom von weniger als 2 %, bevorzugt weniger als 0,1 %, besonders bevorzugt weniger als 0,01 % der Zellen integrieren. Solche Vektoren sind von Vektoren mit höheren Integrationsraten, beispielsweise von Adeno-Associated-Virus (AAV) abgeleitete Vektoren, zu unterscheiden. Bevorzugt sind nicht integrierende Vektoren viraler Herkunft, beispielsweise Vektoren, die aus folgenden Viren abgeleitet worden sind: Epstein-Barr-Virus (EBV), Simian Virus 40 (SV40), BK-Virus, JC-Virus, Adenovirus. Im Falle viraler Vektoren erfolgt das Einbringen bevorzugt über Infektion mit den entsprechenden Viren. Wesentlich ist dabei, dass die Viren nicht zur selbständigen Vermehrung in der Wirtszelle in der Lage sind. Dies kann durch Mutation oder Deletion der entsprechenden Gene (EBNA1 bei EBV; Large T-Antigen bei SV40, BK-Virus und JC-Virus; E1-Gen bei Adenovirus) erreicht werden.

In vivo können rekombinante virale DNA-Konstrukte über Wochen bis Monate im Herzmuskel verbleiben. Im Rahmen des vorliegenden Zellkultur-Verfahrens wurde gefunden, dass auf viralen, insbesondere adenoviralen, Vektoren enthaltene Selektionskonstrukte nicht in das Genom integrieren, sondern dass die Selektionskonstrukte wieder aus den Zellen verschwinden bzw. abgebaut werden. Es wurde ein Zeitfenster gefunden, das ausreicht, um eine vollständige Selektion durchzuführen, aber dennoch kurz genug ist, um einen rechtzeitigen Abbau des Selektionskonstruktes sicher zu stellen.

Von den Adenovirus-Vektoren können alle heute bekannten Vektoren, die kein funktionsfähiges E1-Gen enthalten (in der Regel ist E1 in diesen Vektoren deletiert), im Rahmen der Erfindung verwendet werden. Fast alle bekannten Adenovirus-Vektoren sind in E1 und in E3 deletiert. Ferner gibt es auch in E1, E3 und E4 deletierte Vektoren und so genannte "gutless" Vektoren, denen weitere virale Gene fehlen. Im Rahmen der Erfindung werden bevorzugt Vektoren ohne funktionsfähige E1, E3 und E4-Gene verwendet. Besonders bevorzugt werden Vektoren ohene funktionsfähige E1 und E3-Gene verwendet. Ganz besonders bevorzugt wird der Adenovirus-Vektor Ad5 verwendet. Wesentlich ist, dass die Adenovirus-DNA nicht in das Genom der infizierten Zellen integriert, d.h. im Hinblick auf Integrationsfähigkeit modifizierte adenovirale Konstrukte können nicht verwendet werden.

Bei Infektion mit viralen Vektoren ist es wichtig, dass die entsprechenden Viren in der Lage sind, die verwendeten Ausgangszellen oder die kardiomyogenen Zellen oder Herzmuskelzellen zu infizieren. Dem Fachmann sind Verfahren bekannt, dies festzustellen. Beispielsweise lassen sich hämatopoetische Stammzellen, aber auch primäre (d.h. direkt aus dem Spender in Kultur genommene) Fibroblasten und primäre glatte Muskelzellen schlecht mit Adenoviren infizieren. Hingegen lassen sich beispielsweise mesenchymale Stammzellen, bevorzugt adulte mesenchymale Knochenmarkstammzellen, im Rahmen des erfindungsgemäßen Verfahrens sehr gut mit rekombinanten Adenoviren infizieren. Ferner wurde gefunden, dass sich auch in die kardiomyogene Richtung andifferenzierte Stammzellen, insbesondere mit 5'-Azacytidin andifferenzierte mesenchymale Knochenmarkstammzellen, sehr gut mit rekombinanten Adenoviren infizieren lassen. In allen Fällen konnten mehr als 95% der Zellen erfolgreich infiziert werden, in der Regel mehr als 99%.

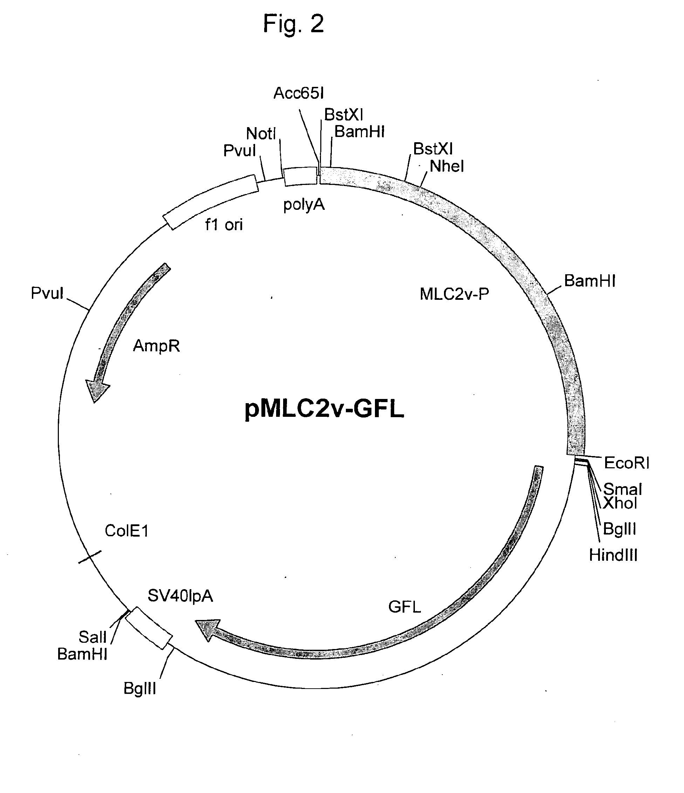

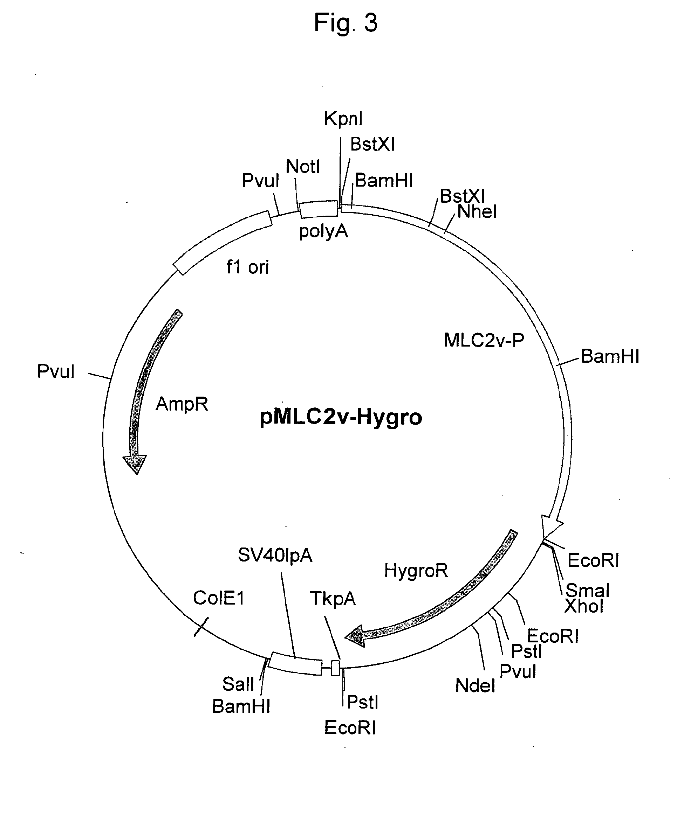

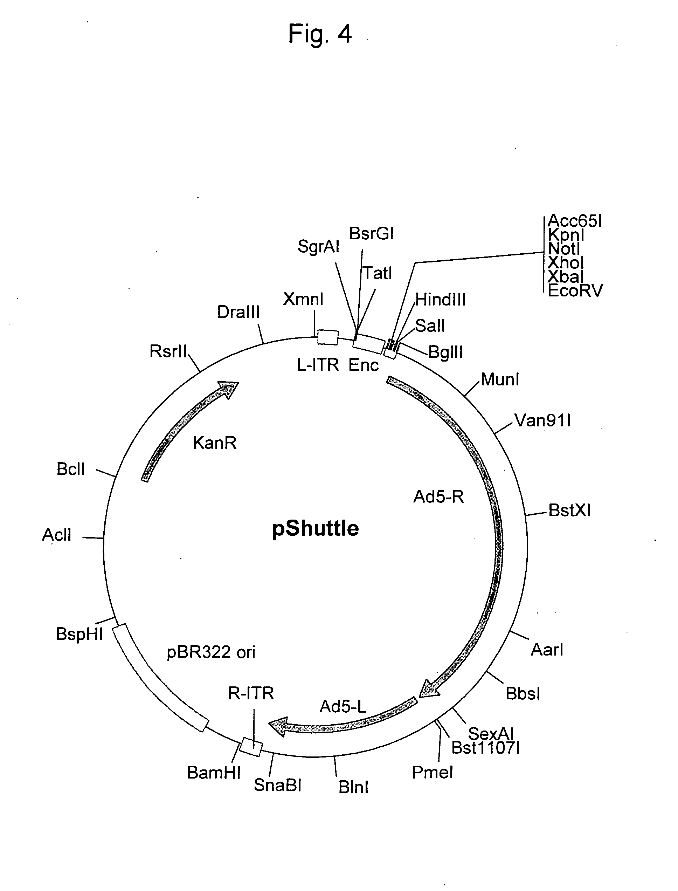

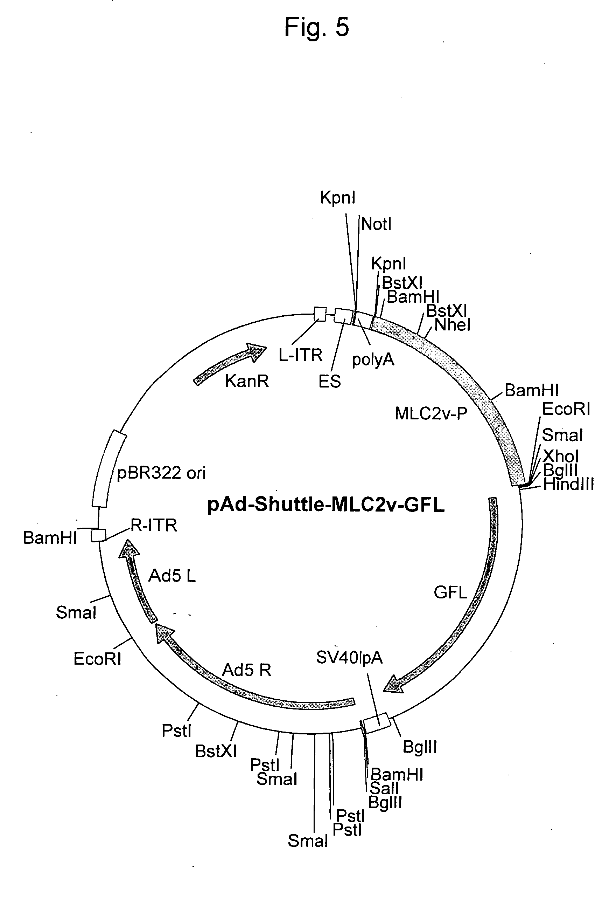

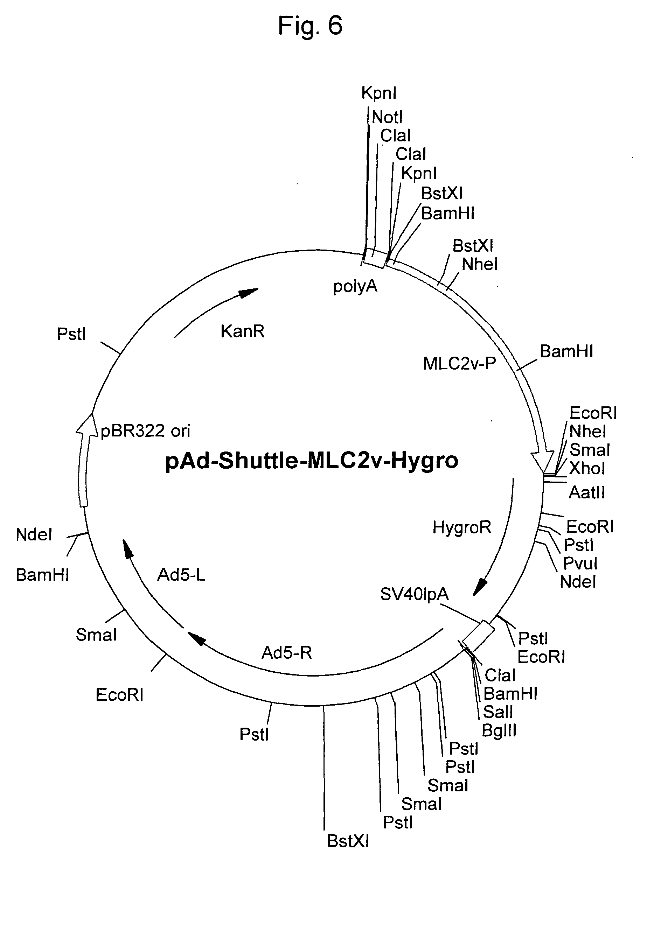

Das Selektionskonstrukt enthält im wesentlichen zwei Komponenten, nämlich Expressionssequenzen und Gene für Marker, bevorzugt Selektionsmarker. Daneben können weitere dem Fachmann geläufige Komponenten enthalten sein, beispielsweise Polyadenylierungssequenzen. Insbesondere können die in den Fig. 2 bis 6 dargestellten Komponenten enthalten sein.

Expressionssequenzen werden im Rahmen der Erfindung in den Konstrukten verwendet, um die Expression eines Markers bzw. Selektionsmarkers, der von dem Konstrukt codiert wird, zu vermitteln. Der Begriff der Expressionssequenzen im Sinne der vorliegenden Erfindung schließt Promotoren, Upstream Elements, Enhancer und Response-Elemente ein. Expressionssequenzen können in zwei Hauptklassen eingeteilt werden, nämlich Promotoren und Enhancer. Ein Promotor im Sinne der vorliegenden Erfindung ist eine DNA-Sequenz, die in relativ festgelegter Position stromaufwärts von der Startstelle der Transkription ihre Aufgabe erfüllt, nämlich die Expression eines Genes zu regulieren. Ein Promotor enthält Core-Elemente, die für die grundlegende Interaktion der RNA-Polymerase und der Transkriptionsfaktoren benötigt werden, aber er kann auch Upstream-Elemente und Response-Elemente enthalten. Der Begriff des Enhancers bezieht sich im Sinne der Erfindung auf eine DNA-Sequenz, die in nicht festgelegtem Abstand von der Stelle der Transkription und in nicht festgelegter Orientierung ihre Aufgabe erfüllt, nämlich dass die Transkription von nahegelegenen Promotoren verstärkt wird. Enhancer und Promotoren können auch Response-Elemente enthalten, die die Regulation der Transkription vermitteln. Als Promotoren bekannte Sequenzen enthalten häufig auch korrekterweise als Enhancer zu bezeichnende Sequenzen. Auch solche Promotoren sind Expressionssequenzen im Sinne der vorliegenden Erfindung.

Der Begriff der Expression bezieht sich im Sinne der Erfindung sowohl auf das vom Gen abgelesene mRNA-Transkript als auch auf das Genprodukt (Protein). Üblicherweise besteht in Eukaryoten eine gute Korrelation zwischen mRNA und der Menge an translatiertem Protein. Dem Fachmann ist es möglich, die Expression zu steigern oder zu verringern, beispielsweise durch Einfügen von Sequenzen, die die mRNA stabilisieren (z.B. SV-40 Polyadenylierungssignal) oder von Sequenzen, die die Translation steigern (z.B. Kozak-Sequenz). Entscheidend im Sinne der vorliegenden Erfindung ist, dass das Genprodukt, insbesondere der Selektionsmarker, in hinreichender Menge zur Verfügung gestellt wird.

Im Rahmen der Erfindung sind solche Expressionssequenzen bevorzugt, die spezifisch in kardiomyogenen Zellen oder in Herzmuskelzellen aktiv sind. Besonders bevorzugt sind Expressionssequenzen, die in spezifisch in kardiomyogenen Zellen aktiv sind, wie sie durch Differenzierung mesenchymaler Stammzellen durch Behandlung mit 5'-Azacytidin erzeugt werden können. Besonders bevorzugt ist der MLC-2v-Promotor. Bevorzugt sind Expressionssequenzen aus Säugetieren, besonders bevorzugt aus Primaten und Nagetieren, ganz besonders bevorzugt aus Mensch.

Spezifische Aktivität bedeutet in diesem Zusammenhang, dass die Expression des von der Expressionssequenz aktivierten Markers in spezifischen Organen, Zell- oder Gewebetypen höher ist als in einer Mehrzahl anderer Organe, Zell- oder Gewebetypen. Gleichermaßen kann sich spezifische Aktivität auch auf bestimmte Entwicklungs- oder Differenzierungsstadien beziehen. Unter höherer Expression ist in diesem Zusammenhang eine mindestens 3-fach, bevorzugt mindestens 10-fach, besonders bevorzugt mindestens 20-fach, ganz besonders bevorzugt mindestens 100-fach erhöhte Expression zu verstehen. Insbesondere soll im Rahmen der Erfindung die Aktivität der Expressionssequenzen in kardiomyogenen Zellen oder Herzmuskelzellen höher sein als die Expression in den Ausgangszellen, z.B. den adulten mesenchymalen Stammzellen. Expression in anderen Organen, Zell- oder Gewebetypen bzw. Entwicklungs oder Differenzierungsstadien kann tolerabel sein, sofern die Expression innerhalb der kardiomyogenen oder Herzmuskelzellen höher ist als in den anderen Zellen, von denen die kardiomyogenen oder Herzmuskelzellen ausselektiert werden sollen.

Spezifische Aktivität in kardiomyogenen Zellen oder Herzmuskelzellen kann auch durch die Deletion solcher Enhancer-Elemente oder Response-Elemente aus den Expressionssequenzen erreicht werden, welche die Expression in anderen Zelltypen betreiben.

Weiterhin bevorzugt sind solche Expressionssequenzen, die spezifisch in differenzierten Herzmuskelzellen aktiv sind. Solche Expressionssequenzen sind aus publizierten Expressionsanalysen bekannt oder können aus den Beispielen 6 bis 12 ersehen werden.

Beispiele für geeignete Expressionssequenzen sind die Promotoren der Gene für α-Myosin-Heavy-Chain (spezifisch für Herzmuskelzellen), MLC-2v (spezifisch für ventrikuläre Herzmuskelzellen), GATA-4, Cardiac Troponin I, Serca 2a, kardiales sarcomeres α-Actin, Nkx 2.5 und Myogenin.

Bevorzugt sind die Promotoren der in den Beispielen 6 bis 12 untersuchten Gene sowie der MLC-2v Promotor. Besonders bevorzugt ist der MLC-2v-Promotor.

Bevorzugt sind Expressionssequenzen, die spezifisch sowohl in kardiomyogenen Zellen, als auch in Herzmuskelzellen aktiv sind. Ein besonders bevorzugtes Beispiel dafür ist der MLC-2v-Promotor.

Um die Expression von den Promotoren zu steigern, können sie auch in Kombination mit Enhancem verwendet werden. Bevorzugt ist der Cytomegalievirus-Enhancer (CMV-Enhancer), der die Expression von diese Promotoren steigern kann, insbesondere vom MLC-2v-Promotor.

Wichtig für das Verfahren ist es, dass die verwendeten Expressionssequenzen in denjenigen anderen Zellen, aus denen die kardiomyogenen und Herzmuskelzellen selektiert werden sollen, nicht aktiv sind. Dem Fachmann ist bekannt, wie die Aktivität untersucht werden kann (beispielswiese durch Antikörper-Färbungen, Polymerase-Kettenreaktion, Northern Blotting, In-Situ-Hybridisation und Hybridisierungen an DNA-Chips). Beispielsweise wurde in mesenchymalen Stammzellen keine endogene Expression herzmuskelspezifischer Gene (α-Myosin-Heavy-Chain, MLC-2v, GATA-4, Cardiac Troponin I, Serca 2a, kardiales sarcomeres α-Aktin gefunden, siehe auch Beispiele 6 bis 12) was zeigt, dass die zugehörigen Expressionssequenzen dort nicht aktiv sind und für das Selektionsverfahren verwendet werden können.

Unspezifische Expression von auf den Vektoren gelegenen sogenannten kryptischen Promotoren kann dadurch vermieden werden, dass vor die Expressionssequenz, insbesondere vor Promotoren, eine Polyadenylierungssignalsequenz eingefügt wird (siehe auch Fig. 2 bis 3 und Fig. 5 bis 6).

Als besonders geeignet wurde der MLC-2v-Promotor gefunden, da er erst nach Behandlung der mesenchymalen Stammzellen mit differenzierungsauslösenden Substanzen, z.B. mit der Substanz 5'-Azacytidin, aktiviert wird, und da diese Aktivierung durch die Differenzierung zu kardiomyogenen Zellen erfolgt. Damit ist der MLC-2v Promotor bereits in einem besonders frühen Differenzierungsstadium aktiv, d.h. noch bevor die kardiomyogenen Zellen kontraktil werden. Daher ist der MLC-2v Promotor für eine Selektion in diesem frühen Differenzierungsstadium geeignet. Das ist vorteilhaft, da frühe Differenzierungsstadien vermutlich über eine besonders gute Integrationsfähigkeit nach Transplantation verfügen. Ferner ist dadurch die Behandlungsdauer für Differenzierung und Selektion verkürzt.

Als zweite wesentliche Komponente enthält das Selektionskonstrukt Gene für Marker, bevorzugt Gene für Selektionsmarker.

Als Marker im Sinne der vorliegenden Erfindung sind alle Genprodukte zu verstehen, deren Expression mit einer Expressionssequenz gesteuert werden kann. Bevorzugt sind unter Markern Reporterproteine (z.B. Luciferase, β-Galaktosidase, GFP einschließlich seiner Derivate wie EGFP, CFP und YFP) und Selektionsmarker zu verstehen. Die einen Marker exprimierenden Zellen können beispielsweise mittels 'Fluorescence-activated cell sorting' (FACS) über die Eigenfluoreszenz des GFP-Proteins oder nach Bindung spezifischer Antikörper, welche direkt mit Fluoreszenz-Molekülen konjugiert bzw. über einen Fluoreszenz-markierten Zweitantikörper detektierbar sind, isoliert werden. Marker können auch Fusionsproteine sein, beispielsweise eine Fusion von GFP und Luciferase. Dem Fachmann ist bekannt, wie solche Fusionsproteine hergestellt werden. Beispielsweise können die kodierenden Sequenzen der zu fusionierenden Proteine im Leseraster mit molekularbiologischen Methoden aneinander kloniert werden.

Selektionsmarker im Sinne der vorliegenden Erfindung sind Genprodukte, die den Zellen, in denen sie exprimiert werden, die Fähigkeit verleihen, unter speziellen Bedingungen zu überleben. Die speziellen Bedingungen werden durch ein geeignetes Selektionsmittel geschaffen. Dabei wird die Expression des Selektionsmarkers von einer geeigneten Expressionssequenz reguliert. Selektionsmarker und Selektionsmittel bilden ein Selektionssystem.
Geeignete Selektionsmittel sind dem Fachmann bekannt. Beispielsweise kann das Selektionsmittel cytotoxisch sein, d.h. nicht resistente Zellen werden unmittelbar abgetötet, es kann aber auch cytostatisch sein, d.h. die Zellen werden zunächst nur an der Vermehrung gehindert und sterben während der weiteren Kultivierung. Auch ohne Absterben der nicht resistenten Zellen kann eine relative Anreicherung der sich weiter vermehrenden kardiomyogenen Zellen oder Herzmuskelzellen und damit das erfindungsgemäße Ziel einer reinen Kultur kardiomyogener Zellen oder Herzmuskelzellen erreicht werden. Beide Arten von Selektionsmitteln sind daher im Rahmen der Erfindung geeignet, wobei cytotoxische Mittel bevorzugt sind. Das Selektionsmittel kann auch ein gegen tierische oder menschliche Zellen wirksames Antibiotikum enthalten. Das Selektionsmittel kann auch aus einem Minimalmedium bestehen, in dem ein oder mehrere essentielle Nährstoffe in einer Form enthalten sind, dass sie nur von Zellen, die den Selektionsmarker exprimieren, verstoffwechselt werden können. Dadurch können wiederum nur die Zellen überleben, die den Selektionsmarker exprimieren. Bekannte Selektionssysteme für eukaryotische Zellen enthalten die folgenden Kombinationen von Selektionsmarkern und Selektionsmitteln:

| Selektionsmarker | Selektionsmittel |
|---|---|
| Neomycin-Phosphotransferase II | z.B. Neomycin, Kanamycin, Paromomycin, Geneticin (G418) |
| Neomycin-Phosphotransferase I | z.B. Neomycin, Kanamycin |
| Hygromycin-Phosphotransferase | z.B. Hygromycin B |
| Blasticidin S Deaminase von Aspergillus terreus | z.B. Blasticidin |
| Puromycin N-acetyl-Transferase | z.B. Puromycin |
| Sh ble von Streptoalloteichus Hindustanus | z.B. Zeocin / Phleomycin |

Im Rahmen der Erfindung ist es wichtig, dass die Selektionssysteme innerhalb eines geeigneten Zeitfensters eine vollständige Selektion erlauben. Das Zeitfenster für eine vollständige Selektion lässt sich durch die Wahl eines geeigneten Selektionssystems, insbesondere des Selektionsmittels, beeinflussen. Beispielsweise ist Hygromycin üblicherweise toxischer als Kanamycin und tötet sensitive Zellen schneller ab. Dabei ist ein äußerer Zeitrahmen allerdings durch die Dauer der Expression des Selektionsmarkers vorgegeben.

Die Selektionssysteme müssen eine Selektion erlauben, während das eingebrachte Selektionskonstrukt in den Zellen vorhanden ist und der Selektionsmarker exprimiert wird. Bevorzugt sind Selektionssysteme, die eine Selektion innerhalb von 21 Tagen, bevorzugt innerhalb von 10 Tagen, besonders bevorzugt innerhalb von 5 Tagen und ganz besonders bevorzugt innerhalb von 4 Tagen nach Einbringen des Selektionsmittels erlauben. Ganz besonders bevorzugt ist die Kombination von Hygromycin-Phosphotransferase und Hygromycin B. Bevorzugte Hygromycin-Konzentrationen sind 10 bis 500 units/ml Hygromycin B, besonders bevorzugt sind 20 bis 100 units/ml, ganz besonders bevorzugt sind 50 units/ml. (units entsprechend Calbiochem Kat-Nr. 400049 zonal inhibition assay mit Bacillus subtilis)

Es ist auch möglich, zwei oder mehr verschiedene Selektionskonstrukte mit unterschiedlichen Expressionssequenzen und unterschiedlichen Selektionssmarkergenen einzubringen. Dadurch lässt sich die Spezifität der Selektion und ggf. auch die Selektivität für bestimmte Zelltypen oder Differenzierungsstadien erhöhen.

Das vorliegende Verfahren ist auch dadurch gekennzeichnet, dass die Selektionskonstrukte nicht dauerhaft (d.h. nur transient) in den Zellen verbleiben. Unter nicht dauerhaftem Verbleib ist ein Verbleib für 2 bis 21 Tage, bevorzugt 3 bis 10 Tage, besonders bevorzugt 3 bis 5 Tage und ganz besonders bevorzugt 4 Tage zu verstehen. Unter Verbleib ist ein Verbleib in einem so großen Teil der Zellen und in so großer Kopienzahl zu verstehen, dass das Genprodukt in kardiomyogenen Zellen oder Herzmuskelzellen ausreichend exprimiert wird. Ausreichende Expression bedeutet dabei, dass z.B. GFP in ausreichender Menge für die Sortierung mit FACS produziert wird, oder dass der Selektionsmarker in ausreichender Menge zur Verleihung einer Resistenz gegen das Selektionsmittel exprimiert wird. Insbesondere ist mit transientem Einbringen gemeint, dass es nicht zu stabiler Integration in das Genom der Zielzellen kommt.

Obwohl nicht integrierende Konstrukte, insbesondere virale Vektoren in vivo über mehr als zwei Wochen extrachromosomal als sogenannte Episomen in Herzmuskelzellen oder anderen Zellen verbleiben können, ist ihr Verbleib in den selektionierten kardiomyogenen Zellen oder Herzmuskelzellen nicht dauerhaft. Die kultivierten Zellen werden mit dem Differenzierungsmittel behandelt und der Vektor mit dem Selektionskonstrukt bevorzugt nach 1 bis 14, bevorzugter nach 2-7, besonders bevorzugt nach 3 bis 4 und ganz besonders bevorzugt nach 4 Tagen in die Zellen eingebracht. Bevorzugt erfolgt das Einbringen in Abwesenheit des Differenzierungsmittels. Bevorzugt erfolgt das Einbringen in niedriger Kopienzahl. Beispielsweise betragen im Falle der bevorzugt verwendeten rekombinanten Adenoviren geeignete Infektionsdosen für die Transfektion (das Einbringen) 10 bis 100 infektiöse Einheiten (plaque forming units, pfu) pro Zelle, bevorzugt sind 20 bis 100 pfu/Zelle, besonders bevorzugt sind 50 pfu/Zelle. Es wurde gefunden, dass selbst eine hohe Infektionsdosis pro Zelle (z.B. 1000 pfu/ Zelle) verwendet werden kann, und dennoch die Zahl tatsächlich der in die Zelle aufgenommenen und erfolgreich freigesetzten adenoviralen Selektionskonstrukte so gering ist, dass sie innerhalb weniger Tage weitgehend vollständig abgebaut werden. 1 bis 3 Tage (in der Regel 2 Tage) nach Einbringen des Vektors ist der Vorgang der Markergen-Expression, und gegebenenfalls der Virusgenomfreisetzung, üblicherweise abgeschlossen. Zugabe des Selektionsmittels (beispielsweise Hygromycin) führt dann innerhalb von 21, in der Regel weniger als 10 weiteren Tagen zur vollständigen Abtötung derjenigen Zellen, die keine Expression des Selektionsmarkers aufweisen, während die kardiomoygenen Zellen oder Herzmuskelzellen in dieser Zeit weiter proliferieren. Das Selektionsmittel muss daher abgesetzt werden, bevor die aufgenommenen Selektionskonstrukte wieder verloren gegangen sind, da sonst auch die Herzmuskelzellen absterben bzw. an der Vermehrung gehindert werden.

Der Zeitpunkt für das Absetzen des Selektionsmittels kann durch dem Fachmann bekannte einfache Tests bestimmt werden. Beispielsweise kann ein dem Selektionskonstrukt entsprechendes DNA-Konstrukt eingebracht werden, das statt des Selektionsmarker-Genes das Gen für GFP oder ein anderes Reporterprotein enthält. Aus der Dauer der Expression des Reportergenes kann dann die Dauer des Verbleibs des Selektionskonstruktes abgeschätzt werden. Üblicherweise muss im vorliegenden Verfahren das Selektionsmittel nach 2 bis 21 Tagen, bevorzugt nach 3 bis 10 Tagen, bevorzugter nach 3 bis 5 Tagen, besonders bevorzugt nach 4 Tagen wieder abgesetzt werden. Der Verlust der Selektionskonstrukte kann durch weitere Verdoppelungen der Zellen (Verdoppelung etwa alle 1 bis 2 Tage) zusätzlich beschleunigt werden. Es sind in der Regel keine Passagen der Zellen notwendig, d.h. die Zellen werden entsprechend dünn ausgesät (1 x 10² bis 1 x 10⁵ Zellen/cm², bevorzugt 1 x 10³ bis 1 x 10⁵ Zellen/cm², besonders bevorzugt 3 x 10³ Zellen/cm²), so dass sowohl die Andifferenzierung der Zellen, als auch das Einbringen des Selektionskonstruktes (bevorzugt über rekombinante Adenoviren), als auch die anschließende Selektion ohne Passagierung durchgeführt werden kann. Um aber größere Mengen kardiomyogener Zellen oder Herzmuskelzellen zu erhalten, kann es sinnvoll sein, die Zellen dichter auszusäen, um sie dann während der Andifferenzierung oder Selektionsperiode zu passagieren. Dabei sind Verdünnungen von 1:2 bis 1:10 bevorzugt. Die Zellen werden entsprechend auf doppelt bzw. zehnmal so viele Zellkulturschalen verteilt. Alternativ können zur Herstellung größerer Mengen von Zellen die Zellen auch nach der Selektion noch weiter kultiviert und vermehrt werden. Die Dauer der Weiterkultivierung bzw. die Zahl der Zellpassagen richtet sich nach der Menge an benötigtem Zellmaterial. Bevorzugt ist eine Weiterkultivierung für 1 bis 12 Wochen, besonders bevorzugt für 4 bis 8 Wochen. Bevorzugt ist eine Zahl von mindestens zwei Zellpassagen, die jeweils durchgeführt werden, wenn die Zellen konfluent geworden sind. Dabei sind Verdünnungen von 1:2 bis 1:10 bevorzugt.

Der Verlust des Selektionskonstruktes kann auch durch Modifikation des Selektionskonstruktes sichergestellt werden: Dabei kann als weiterer Bestandteil in das Selektionskonstrukt hinter dem Selektionsmarker-Gen z.B. eine IRES (intemal ribosomal entry site) eingeführt werden und dahinter das Gen für einen sensitisierenden Marker. Eine weitere Möglichkeit besteht darin, das Gen für den sensitisierenden Marker im selben Leseraster an das Gen für den Selektionsmarker zu fusionieren. Es wird dann ein Fusionsprotein in den Zellen hergestellt, welches beide Funktionen trägt, d.h. Selektionsmarker und sensitisierender Marker.

Damit wird der sensitisierende Marker von den Zellen exprimiert, die auch den Selektionsmarker exprimieren. Ein sensitisierender Marker sorgt dafür, dass Zellen, die diesen Marker exprimieren, gegenüber einem entsprechenden Selektionsmittel sensitisiert werden. Eine geeignete Kombination ist z.B. die Herpes Simplex Virus Thymidinkinase als sensitisierender Marker und Ganciclovir als Selektionsmittel. Mit Zugabe des entsprechenden Selektionsmittels können also alle Zellen abgetötet werden, in denen das Selektionskonstrukt noch verblieben war.

Durch die offenbarten Maßnahmen kann daher sowohl eine Insertionsmutagenese als auch eine dauerhafte Expression der Marker vermieden werden.

Dem Fachmann sind Methoden bekannt, den Verlust des Selektionskonstruktes nachzuweisen. Beispielsweise kann der Verlust des Selektionskonstruktes dadurch nachgewiesen werden, dass zum fraglichen Zeitpunkt das Selektionsmittel zu einer Probe der zu untersuchenden Zellen zugegeben wird. Eine Probe beeinhaltet eine mindestens so große Zahl von Zellen, dass sie für die entsprechende Kultur repräsentativ ist. Andererseits sollten genügend Zellen in der entsprechenden Kultur verbleiben. Bevorzugt enthält die Probe zwischen 10⁶ und 10⁸ Zellen, besonders bevorzugt zwischen 10⁷ und 0.5x10⁸ Zellen. Unter vollständigen Verlust bzw. Abbau im Sinne der Erfindung ist zu verstehen, dass nach Zugabe des Selektionsmittels bevorzugt weniger als 1%, bevorzugter weniger als 0.1%, besonders bevorzugt weniger als. 0.05% und ganz besonders bevorzugt weniger als 0.01% der Zellen überleben.

Der Nachweis des Abbaus des Selektionskonstruktes kann auch mit PCR (Polymerase-Kettenreaktion) oder nested PCR (PCR, bei der zur Erhöhung der Spezifität zwei Amplifikationszyklen mit jeweils unterschiedlichen, geschachtelten (nested) Primerpaaren durchgeführt werden) durchgeführt werden, wobei Primer verwendet werden, die spezifisch an das adenovirale Selektionskonstrukt, bevorzugt die Marker- oder Selektionsmarkergene, binden.

Für das Selektionsverfahren müssen kardiomyogene Zellen oder Herzmuskelzellen zur Verfügung gestellt werden, was bevorzugt dadurch geschieht, dass sie aus geeigneten Ausgangszellen differenziert werden.
Differenzierung im Sinne der vorliegenden Erfindung ist ein Vorgang, bei dem eine Zelle sich zu einem spezialisierten Zelltyp weiterentwickelt. Der spezialisierte Zelltyp muss nicht endgültig ausdifferenziert sein. Beispielsweise kann der spezialisierte Zelltyp auch kardiomyogene Zellen umfassen, die von ausdifferenzierten Herzmuskelzellen zu unterscheiden sind. Transdifferenzierung ist der Vorgang, bei dem eine bereits spezialisierte Zelle sich in eine andersartig spezialisierte Zelle umwandelt. Als ausdifferenziert wird eine Zelle bezeichnet, die sich nicht mehr weiter differenzieren kann und eine spezialisierte Aufgabe ausübt.

Als Ausgangszellen eignen sich sowohl bestimmte Stammzellen als auch bestimmte Nicht-Stammzellen.

Geeignete Nicht-Stammzellen umfassen beispielsweise Fibroblasten und Endothelzellen, welche sich durch Kokultivierung mit Herzmuskelzellen in schlagende Herzmuskelzellen transdifferenzieren lassen. Dabei scheint Zell-Zell-Kontakt oder elektrische Kopplung wesentlich zu sein, so dass keine Überstände aus Herzmuskelzell-Kulturen verwendet werden können.

Bei den Stammzellen können sowohl ES-Zellen als auch adulte Stammzellen verwendet werden. ES-Zellen können aus Embryonen im Morula-Stadium oder aus der inneren Zellmasse von Embryonen im Blastula-Stadium entnommen werden. Die ES-Zellen lassen sich unter Zugabe geeigneter differenzierungshemmender Substanzen (z.B. Leukemia Inhibitory Factor) kultivieren, wobei ihr totipotenter Zustand erhalten bleibt. Sie können sich also weiterhin prinzipiell in alle Zelltypen differenzieren.

Nicht alle Arten adulter Stammzellen sind als Ausgangszellen geeignet, da sich adulte Stammzelltypen nur in jeweils bestimmte Zellarten differenzieren lassen. Im Rahmen der vorliegenden Erfindung sind solche adulten Stammzellen geeignet, die sich zu kardiomyogenen Zellen oder Herzmuskelzellen differenzieren können.

Geeignete adulte Stammzellen sind beispielsweise solche, die aus Knochenmark, periphärem Blut, Nabelschnurblut, Fettgewebe, oder Herzmuskelgewebe gewonnen werden können. Weitere Beispiel umfassen Multipotente Adulte Progenitor Cells (MAPCs), welche eine sehr seltene Population von Knochenmarkstammzellen mit hoher Plastizität (d.h. Entwicklungspotential in unterschiedliche Zelltypen) und sehr langer Zellteilungsfähigkeit darstellen.

Viele der genannten Stammzellen stellen keine einheitlichen Populationen dar. Das erfindungsgemäße Verfahren kann auch zur Selektion der aus solchen uneinheitlichen Populationen differenzierten Zellen dienen. Die adulten Knochenmarkstammzellen umfassen beispielsweise hämatopoetische Stammzellen und mesenchymale Stammzellen (= Stromazellen), wobei sich nur die mesenchymalen Stammzellen zu kardiomyogenen Zellen oder Herzmuskelzellen differenzieren können. Das erfindungsgemäße Verfahren erfordert aber keine vorherige Separierung der adulten Knochenmarkstammzellen.

Bevorzugte adulte Stammzellen im Rahmen der vorliegenden Erfindung sind mesenchymale Stammzellen aus periphärem Blut, Nabelschnurblut und Knochenmark.

Besonders bevorzugt sind adulte Knochenmarksstammzellen, ganz besonders bevorzugt sind mesenchymale Knochenmarksstammzellen.

Dem Fachmann ist bekannt, wie adulte Knochenmarkstammzellen gewonnen werden können. Im kleinen Maßstab können Knochenmarkstammzellen beispielsweise durch Ausspülen mit Hilfe einer Kanüle gewonnen werden. Dabei wird eine kleine Menge (z.B. 0.5-50 ml, bevorzugt 2-10 ml) sterile gepufferte Kochsalzlösung in das Knochenmark injiziert und die Zellen mit der Flüssigkeit in die Spritze gezogen.

Für größere Mengen an Knochenmark kann die Entnahme durch mehrere Punktionen des Beckenkammes (den Schaufeln des Beckens) erfolgen. Bei menschlichen Spendern lassen sich so etwa 1-1,5 Liter Knochenmark, vermischt mit Blut, entnehmen.

Alternativ können adulte Knochenmarkmarkstammzellen auch aus Blut entnommen werden, wobei der Spender zunächst für etwa eine Woche mit Zytokinen wie z.B. G-CSF (Granulocyten - Koloniestimulierender Faktor) behandelt wird. Diese Behandlung erhöht die Zahl der Knochenmarkstammzellen im Blut. Das Verfahren birgt allerdings Gefahren durch die benötigte Zytokin-Behandlung.

Neben den Knochenmarkstammzellen sind bei den entnommenen Zellen also auch immer Blutzellen vorhanden, die abgetrennt werden müssen. Da Blutzellen bei Kultivierung nicht am Boden der Kulturschale oder Kulturflasche anhaften sondern in Suspension verbleiben, können sie durch Wechsel des Kulturmediums von den Knochenmarkzellen leicht getrennt werden. Da die hämatopoetischen Stammzellen nicht anhaftend (adhärent) wachsen, lassen auch sie sich in gleicher Weise entfernen.

Die Erythrozyten, innerhalb der Blutzellen die weitaus größte Population, können auch in einem hypotonen Medium (bevorzugt 20-40 sec. in 2-10 mM EDTA, besonders bevorzugt 30 sec. in 5 mM EDTA) lysiert werden, so dass sich die Knochenmarkzellen durch Zentrifugation abtrennen lassen. Alternativ können die Knochenmarkzellen durch Dichtezentrifugation von Blutzellen getrennt werden.

Die Zellen können nach dem Fachmann bekannten Verfahren kultiviert werden. Geeignet ist beispielsweise das folgende Verfahren. Als Medium wird IMDM (Isocove's modified Dulbecco's medium (Invitrogen)) unter Zusatz von fetalem Kälberserum (bevorzugt 15-25%, besonders bevorzugt 20%) verwendet. Andere geeignete Zellkulturmedien sind beispielsweise kommerziell von CellSystems Biotechnologie Vertrieb GmbH, St. Katharinen, erhältlich. Zur Vermeidung von Kontaminationen ist es sinnvoll, Antibiotika / Antimycotika zum Medium hinzuzugeben. Bevorzugt ist die folgende Kombination: Penicillin (bevorzugt 100 IU/ml), Streptomycin (bevorzugt 100 µg/ml), Gentamycin (bevorzugt 50 µg/ml) and Amphotericin B (bevorzugt 1 µg/ml). Medium und Antibiotika werden im Folgenden als Vollmedium bezeichnet. Bei nachfolgender Transplantation in einen menschlichen Empfänger ist es bevorzugt, humanes Serum zu verwenden, welches auch vom Empfänger stammen kann. Bevorzugt ist ferner die Verwendung serumfreier (synthetischer) Medien, wie sie dem Fachmann bekannt sind.

Die Zellen werden in einer Dichte von bevorzugt 1x10⁵-1x10⁶ cells/cm2, besonders bevorzugt 0,2x10⁶ cells/cm2, in Zellkultur-Petrischalen oder Zellkulturflaschen mit Vollmedium (s.o.) ausgesät. Wie oben beschrieben kann es sinnvoll sein, die adhärenten adulten Stammzellen durch mehrmaliges Ausplattieren auf Petrischalen und Austausch des Mediums von den nicht adhärent wachsenden Zellen zu trennen. Dabei erfolgt das Ausplattieren für bevorzugt mindestens 0,75 h, besonders bevorzugt mindestens 1 h. Die Kulturen werden bevorzugt bei 36-38°C und 4-6% CO₂, besonders bevorzugt bei 37°C und 5% CO₂, inkubiert. Nach 1-4 Tagen, bevorzugt 3 Tagen wird ein Teil (bevorzugt die Hälfte) des Mediums entfernt und durch frisches Vollmedium ersetzt. Dieser Vorgang wird nach weiteren 3-10, bevorzugt 5-10 Tagen, je nach Wachstum der Zellen, wiederholt. In der Regel sind die Zellen nach etwa 14 Tagen konfluent, d.h. sie bedecken den Boden der Kulturschale oder -flasche vollständig. Die Zellen werden mit Hilfe von Trypsin von der Kulturschale gelöst (bevorzugt 0,025% Trypsin/0,1 mM EDTA für 5 min) und zu bevorzugt 1x10⁴ cells/cm² in Zellkulturflaschen ausgesät. Nach 2-3 Passagen (Ablösungen und verdünnten Wiederaussaaten) können die Zellen eingesetzt werden.

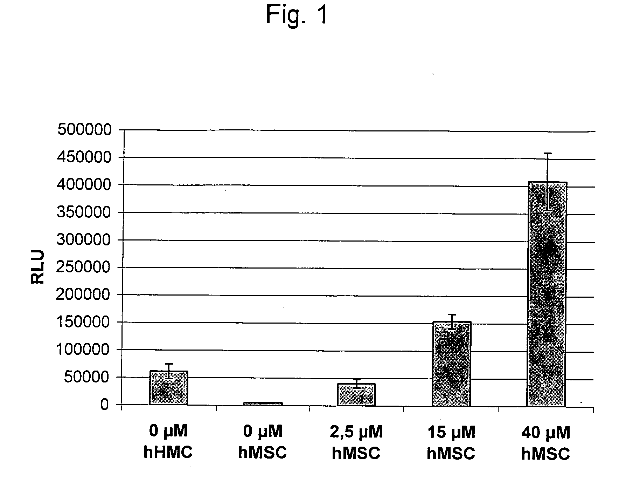

Stammzellen, bevorzugt adulte Stammzellen können auf unterschiedliche Weise zu kardiomyogenen Zellen und/oder Herzmuskelzellen differenziert werden. Geeignete Differenzierungsmittel sind dem Fachmann bekannt. Bedingung für die Eignung der Differenzierungsmittel ist lediglich, dass sie in der Lage sind, die entsprechenden Stammzellen zu kardiomyogenen Zellen oder Herzmuskelzellen zu differenzieren. Bevorzugt ist es, Differenzierungsmittel zu verwenden, die eine Differenzierung zu kardiomyogenen Zellen bewirken, nicht jedoch zu ausdifferenzierten Herzmuskelzellen. Dies kann auch durch entsprechend kurze Einwirkungszeit der Differenzierungsmittel erreicht werden. Grundsätzlich ist die Dauer der Behandlung mit dem Differenzierungsmittel abhängig von dem jeweils eingesetzten Mittel. Die benötigte Dauer der Behandlung und die benötigte Konzentration des Differenzierungmittels kann beispielsweise durch Einbringen eines adenoviralen Konstruktes beurteilt werden, welches GFP oder einen anderen Marker unter Kontrolle eines in kardiomyogenen Zellen oder Herzmuskelzellen spezifisch aktiven Promotors exprimiert (siehe auch Beispiel 2 und Fig. 1).

Die Differenzierungsauslösung erfolgt bevorzugt in vitro. Ein Vorteil von Differenzierungsverfahren in vitro ist, dass auch Differenzierungsmittel eingesetzt werden können, die in vivo zu starken Nebenwirkungen führen könnten oder die sich in vivo nicht effizient einsetzen lassen. Bevorzugt ist die Differenzierung aus adulten Stammzellen, besonders bevorzugt mesenchymalen Stammzellen, ganz besonders bevorzugt mesenchymalen Knochenmarkstammzellen.

Geeignete Differenzierungsmittel umfassen die Verwendung demethylierender Substanzen, die Kokultivierung mit Herzmuskelzellen, Inhibitoren der Histondeazetylierung (z.B. Trichostatin A), Amphotericin B, Dexamethason, Retinsäure und Proteine (Signalproteine und Transkriptionsfaktoren).

Bevorzugte Differenzierungsmittel umfassen die Verwendung demethylierender Substanzen, Inhibitoren der Histondeazetylierung (z.B. Trichostatin A) und Proteine (Signalproteine und Transkriptionsfaktoren).

Bevorzugte demethylierende Substanzen schließen DNA-Methyltransferase-Inhibitoren ein. Besonders bevorzugt sind 5'-Azacytidin und 5-aza-2-deoxizytidin (=Decitabin). Ganz besonders bevorzugt ist 5'-Azacytidin. Bevorzugte Konzentration von 5' Azacytidin ist 1 bis 80 µM, bevorzugter 2.5 bis 40 µM, besonders bevorzugt 5 bis 20 µM, ganz besonders bevorzugt 10 µM. Der Vorteil des Selektionsverfahrens beruht auch darin, dass auch geringe und daher untoxische Konzentrationen von 5'-Azacytidin für eine effiziente Differenzierung genutzt werden können. Bevorzugt ist eine einmalige Gabe von Azacytidin und anschließende Kultivierung der Zellen für 1 bis 14, bevorzugter 2 bis 7, besonders bevorzugt 3-4 und ganz besonders bevorzugt 4 Tage.

Geeignete Proteine sind solche, die in der Lage sind, eine Differenzierung zu kardiomyogenen Zellen oder Herzmuskelzellen auszulösen. Solche Proteine sind dem Fachmann bekannt. Die Proteine können ihre differenzierungsauslösende Wirkung durch Bindung an Zelloberflächenrezptoren entfalten oder sie müssen in die Zellen aufgenommen werden. Proteine, die als Differenzierungsmittel im Sinne der vorliegenden Erfindung ihre Wirkung durch Bindung an Zelloberflächenrezeptoren entfalten, müssen lediglich in das Kulturmedium gegeben werden. Solche Proteine umfassen beispielsweise Activin, TGF-beta, BMP-2, BMP-4 und FGF-4.

Proteine, die als Differenzierungsmittel in die Zellen aufgenommen werden müssen, umfassen beispielsweise MyoD, GATA-4 und Nkx 2.5. Dem Fachmann sind Methoden bekannt, die Proteine mit Hilfe geeigneter Transfektionsmittel direkt in die Zellen einzubringen. Geeignete Transfektionsmittel für das direkte Einbringen umfassen beispielsweise Elektroporation, Liposomen, oder Fusionen der einzubringenden Proteine mit dem HIV-TAT Peptid, VP-22, oder mit Penetratinen.

Dem Fachmann ist es möglich, Modifikationen der genannten Differenzierungsmittel zu entwickeln und somit die Effizienz der Differenzierung zu steigern. Einige Differenzierungsmittel können auch in Kombination eingesetzt werden, beispielsweise kann Trichostatin A in Kombination mit 5'-Azacytidin eingesetzt werden.

Bestimmte Ausgangszellen, z.B. Endothelzellen und mesenchymale Knochenmarkstammzellen, können durch Kokultivierung mit Herzmuskelzellen zu kardiomyogenen Zellen differenziert werden. Besonders im Falle der Kokultivierung ist es empfehlenswert, bereits die Ausgangszellen mit dem Selektionskonstrukt zu transfizieren. So werden die die Differenzierung auslösenden Zellen bei der anschließenden Selektion gemeinsam mit den nicht differenzierten Zellen abgetötet.

Die reinen Kulturen kardiomyogener Zellen oder Herzmuskelzellen können in einen tierischen oder menschlichen Empfänger verpflanzt werden. Bevorzugt ist der tierische oder menschliche Empfänger derselbe, aus dem auch die Ausgangszellen, gewonnen wurden (autologe Transplantation). Bevorzugt ist ein tierischer oder menschlicher Empfänger, der an einem Mangel an Herzmuskelzellen leidet. Bei einem tierischen Empfänger kann zu Versuchszwecken ein Mangel an Herzmuskelzellen künstlich hervorgerufen worden sein. Bei einem tierischen Empfänger kann ein typischer Versuch die folgenden Schritte umfassen: (A) Transplantation der mit dem vorliegenden Verfahren gewonnenen reinen kardiomyogenen Zellen und Herzmuskelzellen, (B) Untersuchung der Verbesserung der Herzmuskelleistung, (C) Histologische Analyse des Herzmuskels.

Statt in einen Organismus (Tier oder Mensch) kann die Verpflanzung der Zellen auch in ein Organ oder Gewebe in Organkultur oder Gewebekultur erfolgen. D.h. auch solche Organe oder Gewebe können als Empfänger im Sinne der vorliegenden Erfindung verstanden werden. Bevorzugt ist das Organ oder Gewebe ein Herz oder aus einem Herzen gewonnen.

Die in den Empfänger verpflanzten Zellen können vor der Transplantation mit einem geeigneten Marker, z.B. einem Fluoreszenzfarbstoff, markiert werden (z.B. CellTracker™ CM-DiI oder mit Fluoreszenzfarbstoffen gekoppeltes Dextran). Auf diese Weise kann die Integration der Zellen in den Gewebeverband analysiert werden. Eine solche Analyse ist hilfreich, um z.B. das Transplantationsverfahren oder das Differenzierungs- und Selektionsverfahren für erfolgreiche Transplantation zu verbessern.

Die mit dem vorliegenden Verfahren gewonnenen Zellen können auch in einen heterologen Empfänger verpflanzt werden, d.h. in einen Empfänger, der mit dem Spender nicht identisch ist.

Bei Transplantation in heterologe Empfänger ist die Immunabwehr des Empfängers für Erfolg oder Misserfolg (Abstoßung der Zellen) entscheidend. Je schwächer die Immunabwehr des heterologen Empfängers, desto größer sind die Aussichten, dass die erfindungsgemäß erzeugten Zellen nicht abgestoßen werden.

Dem Fachmann ist bekannt, wie die Immunabwehr eines Empfängers geschwächt oder unterdrückt (supprimiert) werden kann. Die Immunabwehr eines heterologen Empfängers kann z.B. auf genetische, pathologische, oder medikamentöse Weise supprimiert sein. Genetische Immunsuppression ist z.B. bei Nacktmäusen gegeben. Pathologische Immunsuppression ist beispielsweise bei Patienten mit AIDS (Aquired Immune Deficiency Syndrome) gegeben. Medikamentöse Methoden, die Immunabwehr eines Empfängers gegen heterologe Zellen zu supprimieren, umfassen beispielsweise die Gabe von Cyclosporin A, Rapamycin oder immunsupprimierender Antikörper (z.B. anti-CD-25).

Ferner können die Spender-Zellen so modifiziert werden, so dass sie vom Immunsystem des Empfängers nicht abgestoßen werden. Beispielsweise können die Zellen Rezeptoren exprimieren, die die Immunabwehr gegen diese Zellen supprimieren, wie z.B. CTLA-4 (= CD152).

Je höher die immunologische Übereinstimmung von Spender und Empfänger, desto größer sind die Aussichten, dass die erfindungsgemäß erzeugten Zellen nicht abgestoßen werden. Die immunologische Übereinstimmung ist in erster Linie genetisch festgelegt. Bevorzugt ist der Empfänger daher genetisch identisch (syngen) oder ein Verwandter ersten Grades des Spenders. Besonders bevorzugt sind Spender und Empfänger genetisch identisch.

Der Grad der immunologischen Übereinstimmung kann über Gewebetypisierung bestimmt werden. Dem Fachmann sind entsprechende Methoden zur Gewebetypisierung bekannt. Gewebetypisierungen werden auch von kommerziellen Anbietern durchgeführt. So kann der Fachmann aus einer Bank von gewebetypologisch charakterisierten Kulturen kardiomyogener oder Herzmuskelzellen diejenigen Kulturen auswählen, die im Empfänger die voraussichtlich geringste immunologische Abstoßung erfahren. Gewebetypisierung im Sinne der vorliegenden Erfindung beinhaltet beispielsweise die Untersuchung der Gewebe, vorzugsweise weißer Blutzellen (Leukozyten), auf Histokompatibilitätsantigene (HLA-System, humane Leucocytenantigene). Insgesamt sind mehr als 1300 verschiedene HLA-Allele (Ausprägungsformen der jeweiligen Gene) bekannt. Bei einer Transplantation der erfindungsgemäß selektierten Zellen (bzw. Gewebe aus Gewebekultur) ist auf eine möglichst große Übereinstimmung der Spender- und Empfänger-Allele zu achten. Für eine erfolgreiche Transplantation von kardiomyogenen Zellen und Herzmuskelzellen ist besonders auf eine Übereinstimmung der HLA-A-, -B- und -C-Gene zu achten.

Das vorliegende Erfindung betrifft daher auch Zellkultur-Banken mit reinen Kulturen von kardiomyogenen Zellen und Herzmuskelzellen zu erstellen, die aus Zellen unterschiedlicher Spender gewonnen wurden. Die Kulturen oder die entsprechenden Spender sind vorzugsweise gewebetypisiert worden. Die Verwendung gewebetypischer Kulturen aus einer solchen Bank ist insbesondere vorteilhaft, wenn aufgrund des Krankheitsbildes des Empfängers keine Zeit für eine Gewinnung der sonst bevorzugten autologen kardiomyogenen Zellen und Herzmuskelzellen verbleibt. Bevorzugt umfassen die Banken mindestens 100 Kulturen verschiedener Spender, besonders bevorzugt mindestens 1000, ganz besonders bevorzugt mindestens 10000 Kulturen verschiedener Spender.
Gewebetypisierte reine Kulturen kardiomyogener Zellen oder Herzmuskelzellen können auch aus bereits vorhandenen Banken mit gewebetypisierten Stammzellen (z.B. mesenchymalen Stammzellen aus Nabelschnurblut) mit dem erfindungsgemäßen Verfahren gewonnen werden.

Der heterologe Empfänger kann auch anderen Spezies angehören als der Spender, er kann also xenogen sein. In diesem Falle ist es bevorzugt, wenn der Spender genetisch oder gentechnisch verändert worden ist, so dass vom Spender gewonnene Zellen vom Immunsystem des Empfängers nicht oder für eine dauerhafte Abstoßung unzureichend erkannt werden. Bevorzugt betrifft die genetische oder gentechnische Veränderung die Einführung von immunsuppressiven Rezeptoren (z.B. CTLA-4 (= CD152)) oder die Mutation bzw. Deletion von HLA-Genen. Besonders bevorzugt betrifft die genetische oder gentechnische Veränderung daher die Mutation oder Deletion von HLA-Genen. Bevorzugt ist der xenogene Spender ein nicht-menschliches Säugetier, besonders bevorzugt ein Schwein oder nicht-menschlicher Primat. Bevorzugt ist der Empfänger ein Mensch oder ein nicht-menschlicher Primat.

Die mit dem erfindungsgemäßen Verfahren hergestellten Zellen lassen sich auch zur Herstellung von Gewebe (Tissue Engineering) einsetzen. Dem Fachmann sind entsprechende Methoden bekannt. Beispielsweise wird eine geeignete Gewebekultur-Matrix aus biologischem oder nicht-biologischem Material mit den selektierten kardiomyogenen Zellen oder Herzmuskelzellen ex vivo zusammengebracht. Die Matrix wird von den Zellen besiedelt, die nach Kultivierung einen geschlossenen Gewebeverband bilden. Die Gewebekultur-Matrix ist bevorzugt dreidimensional, so dass beispielsweise ein zu ersetzendes Gewebestück, bevorzugt ein Teil des Herzmuskels, bereits in Kultur (ex vivo) nachgebildet wird. Unter geschlossenem Gewebeverband ist im Rahmen der vorliegenden Erfindung zu verstehen, dass die Zellen über Zell-Zell-Kontakte miteinander verbunden sind. Die besiedelte Gewebekultur-Matrix, oder Teile davon, kann dann in den Empfänger implantiert werden, wobei die eingesetzte Matrix üblicherweise nach und nach von einer sich natürlich bildenden extrazellulären Matrix und Stützgewebe ersetzt wird. Um einen natürlichen Gewebeverband zu bilden, können zur Besiedlung der Gewebekultur-Matrix noch andere Zelltypen als kardiomyogene Zellen oder Herzmuskelzellen aufgebracht werden, beispielsweise Bindegewebszellen.

Die Transplantion kann auch in Form eines Arzneimittels erfolgen, wobei das Arzneimittel kardiomyogene Zellen oder Herzmuskelzellen aus einer reinen Kultur in einem geeigneten pharmazeutischen Träger enthält. Dem Fachmann sind Verfahren zur Herstellung solcher Arzneimittel bekannt. Beispielsweise werden die Zellen nötigenfalls von Zelltrümmern, Kulturmedium und evtl. vorhandenen schädlichen Zusätzen wie Glycerin gereinigt. Danach werden die Zellen in den pharmazeutischen Träger, z.B. in eine physiologische Kochsalzlösung, überführt, wobei eine angemessene Konzentration von Zellen in der Suspension eingestellt wird (bevorzugt 10⁵-10⁸ Zellen/ml, besonders bevorzugt 10⁷ Zellen/ml). Der pharmazeutische Träger kann weitere Substanzen enthalten, insbesondere solche, die geeignet sind, die Zellen zu stabilisieren. Die Zellen werden auf geeignete Weise in den Herzmuskel eingebracht, beispielsweise durch direkte Injektion, bevorzugterweise an eine geschädigte Stelle, oder über einen Zugang in den Blutstrom des Herzens. Die Zahl der eingebrachten Zellen ist dabei abhängig von der Größe der geschädigten Stelle. Gegebenfalls muss das Einbringen der Zellen wiederholt werden, bis die geschädigte Stelle hinreichend verheilt ist.

Die selektierten Zellen können auch bis zu ihrer Verwendung gelagert bzw. konserviert werden. Das hat den Vorteil, dass die Zellen leichter versendet werden können. Zweitens kann durch entsprechende Lagerung bzw. Konservierung eine Dedifferenzierung der Zellen durch zu lange Zellkultur vermieden werden. Drittens kann eine zu transplantierende Zellsuspension direkt aus einer gelagerten Suspension hergestellt werden. Das ist besonders vorteilhaft, wenn die Zellen im Rahmen einer Bank von gewebetypisch charakterisierten Kulturen kardiomyogener Zellen oder Herzmuskelzellen verwendet werden sollen, denn dabei kann erhebliche Zeit vergehen, bis für eine gegebene Kultur ein gewebetypisch hinreichend übereinstimmender Empfänger aufgetreten ist.

Dem Fachmann sind geeignete Lagerungs- und Konservierungsmethoden für kultivierte Zellen bekannt, beispielsweise Einfrieren, Trocknung und Gefriertrocknung (Lyophilisierung). In einem bevorzugten Beispiel wird zunächst die Bindung der Zellen an die Kulturschale durch Zusatz von Enyzmen (z.B. Trypsin) und EDTA von der Kulturschale gelockert. Das trypsinhaltige Medium wird entfernt und die Zellen werden von der Schale gewaschen und, ggf. nach weiteren Waschungen, resuspendiert. Danach werden die Zellen unter Zusatz von Dimethylsulfoxid (DMSO) oder Glycerin in flüssigem Stickstoff eingefroren. Dabei kann ein Zusatz von Trehalose die Beständigkeit der Zellen erhöhen. Die Zellen können in gefrorenem Zustand auch leicht versendet werden. Zur Wiedergewinnung der Zellen werden sie beispielsweise in einem Wasserbad bei 37 Grad Celsius rasch aufgetaut. Die aufgetauten Zellen können wieder in Kultur genommen oder auch direkt zur Herstellung eines Arzneimittels verwendet werden.

Eine andere Möglichkeit zur Lagerung oder zum Versand von Zellen besteht in der schonenden Trocknung unter Vakuum, bevorzugt unter Zugabe von Glycerol oder Trehalose. Auch eine Gefriertrocknung (Lyophilisierung) in Gegenwart von Glycerol oder Trehalose kann durchgeführt werden.

Statt der bevorzugten erfindungsgemäßen DNA-Konstrukte können auch DNA-Konstrukte mit alternativen Sequenzen verwendet werden. Die alternativen Sequenzen betreffen im Wesentlichen gleiche Sequenzen mit im Wesentlichen gleicher biologischer Funktion. Unter wesentlicher biologischer Funktion wird bei Expressionssequenzen die Fähigkeit verstanden, spezifisch in kardiomyogenen Zellen oder Herzmuskelzellen aktiv zu sein. Unter wesentlicher biologischer Funktion wird bei Selektionsmarkern die Fähigkeit verstanden, Resistenz gegen das entsprechende Selektionsmittel oder ein strukturell verwandtes Selektionsmittel zu verleihen. Unter wesentlicher biologischer Funktion wird bei Vektoren die Fähigkeit verstanden, in die Ausgangszellen, in kardiomyogene Zellen, oder in Herzmuskelzellen eingebracht werden zu können und nicht stabil in das Genom zu integrieren.

Dem Fachmann sind Methoden geläufig, geeignete alternative Sequenzen zu finden oder artifiziell zu erzeugen. Artifizielle Sequenzen sind durch molekularbiologische Methoden aus natürlichen Sequenzen abgeleitet worden. Dies umfasst Insertionen, Deletionen und Punktmutationen. So können Promotorsequenzen verkürzt, verlängert oder verändert werden, ohne dass sich ihre wesentliche biologische Funktion verändert. Kodierende Sequenzen können z.B. durch stille Mutationen, die auf der Degenerierung des genetischen Codes beruhen, verändert werden.

Geeignete alternative Sequenzen können auch durch natürliche Selektion entstanden sein. So ist es dem Fachmann geläufig, homologe Expressionssequenzen oder Selektionsmarker aus verwandten Spezies zu klonieren. Ebenfalls können verwandte Stämme von viralen Vektoren gefunden werden. Je näher verwandt die Spezies oder der Stamm, desto höher ist die Wahrscheinlichkeit, dass die entsprechenden Sequenzen in äquivalenter Weise verwendet werden können. Für Säugetier-Expressionssequenzen sind bevorzugt Expressionssequenzen aus anderen Wirbeltieren, besonders bevorzugt aus anderen Säugetieren verwendbar. Homologe Sequenzen verwandter Spezies können z.B. über molekularbiologische Verfahren Hybridisierung mit markierten Proben oder PCR mit degenerierten Primern, oder über Datenbankrecherchen ermittelt werden. Die Homologie innerhalb von Expressionssequenzen ist häufig auf kurze Abschnitte begrenzt. Daher können zunächst auch die homologen Gene (z.B. MLC-2v) über Hybridisierung oder Datenbankrecherchen ermittelt werden. Ausgehend von den Genen ist es dann möglich, die angrenzenden Expressionssequenzen über Standardmethoden der Molekularbiologie oder Datenbankrecherchen zu ermitteln.
Durch Sequenzvergleiche verschiedener natürlicher Sequenzen lassen sich stark oder weniger stark konservierte Sequenzabschnitte unterscheiden. Stark konservierte Sequenzabschnitte können üblicherweise nur wenig verändert werden, ohne dass die wesentliche biologische Funktion beeinträchtigt wird. Unter stark konservierten Bereichen werden Bereiche verstanden, die mindestens 40 %, bevorzugt mindestens 60 %, besonders bevorzugt mindestens 70 % identische Aminosäuren aufweisen. Schwach oder gar nicht konservierte Bereiche können hingegen in der Regel verändert werden, ohne dass die wesentliche biologische Funktion beeinträchtigt wird. Daher können Sequenzvergleiche im Hinblick auf die wesentliche biologischen Funktion charakterisierter Sequenzen Informationen liefern, welche Sequenzbereiche verändert werden können, ohne dass ein Verlust der wesentlichen biologischen Funktion in Kauf genommen werden muss. In gleicher Weise können Sequenzen auch optimiert werden, z.B. indem Sequenzen entfernt werden, die die wesentliche Funktion einschränken. Bei Expressionssequenzen kann es sich beispielsweise um die Entfernung von Repressor-Elementen handeln.

Dem Fachmann sind verschiedene Methoden zur Herstellung rekombinanter Adenoviren bekannt.

Eine geeignete Methode beinhaltet die Klonierung des interessierenden Genes in einen Shuttlevektor und die Übertragung dieses Genes in das Adenovirusgenom durch homologe Rekombination in Zellkulturen. Die Isolation und Identifizierung von rekombinanten Adenoviren über diese Methode beinhaltet mehrfache Isolationen von viralen Plaques und ist mühsam und zeitaufwendig.

Als bevorzugte Methode ist dem Fachmann ein homologes Rekombinationssystem bekannt, in welchem die homologe Rekombination in Escherichia coli-Stämmen durchgeführt wird, die über hohe Rekombinationsfähigkeiten und hohe Transformationseffizienz verfügen. Geeignet ist z.B. der E. coli Stamm BJ5183. Das System ist kommerziell erhältlich, z.B. in Form von Stratagenes AdEasy-System. In dem AdEasy-System wird die interessierende DNA-Sequenz in einen Shuttlevektor, pShuttle, einkloniert. Dieser Shuttlevektor wird linearisiert und mit dem viralen DNA-Plasmid in dem Stamm BJ5183 co-transformiert. Die Tranformanten können auf Kanamycin selektiert werden und rekombinanten adenovirale Plasmide können über Restriktionsverdaus identifiziert werden. Die rekombinanten adenoviralen Plasmide können im großen Maßstab über rekombinations-defiziente Bakterien, die für große Konstrukte geeignet sind, produziert werden. Geeignete Bakterien sind z.B. die E. coli Bakterienstämme HB101, DH10B und XL10-Gold.

Zur Herstellung von Viren werden die rekombinanten adenoviralen Plasmide gereinigt und mit geeigneten Restriktionsenzymen linearisiert, so dass die Inverted Terminal Repeats (ITR) exponiert werden. Die besagte linearisierte DNA wird in geeignete Zellen transfiziert, welche diejenigen viralen Gene exprimieren, die den rekombinanten Adenovirusvektoren fehlen und welche für die Herstellung des Virus benötigt werden. Geeignete Zelllinien sind z.B. HEK293, 911, 911E4, PERC6 und 10SW. Die 293-, 911-911E4 und PERC6-Zellen exprimieren die E1 Genprodukte, welche für die Vermehrung aller rekombinanten Adenoviren benötigt werden, wobei die 911E4-Zellen zusätzlich auch die E4-Genprodukte exprimieren.

Für die Transfektion der Zelllinien können alle bekannten Transfektionsmethoden verwendet werden, die zur Transfektion entsprechend großer DNA-Konstrukte geeignet sind. Beispielsweise kann die Transfektion über Calcium-Phosphat-Präzipitation erfolgen. Nach Lyse der tranfizierten Zellen können die ins Medium entlassenen Viren zur Infektion weiterer Zellen verwendet werden. Diese weitere Infektion ist bevorzugt, da dadurch ein Virusstock mit hohem Titer (bevorzugt 10⁸-10¹² plaque forming units/ml) erzeugt werden kann. Plaque forming units (pfu) beschreibt die Zahl der Plaques, die von den in einem bestimmten Volumen enhaltenen Viren auf einem Rasen von Wirtszellen ausgelöst werden.

Dem Fachmann sind Methoden zur Gewinnung gereinigter Viruspartikel bekannt. In einem bevorzugten Verfahren werden die mit Adenoviren infizierten Zellen nach Auftreten des durch die Viren hervorgerufenen cytopathischen Effektes (CPE) abzentrifugiert und mit einem Glashomogenisator aufgeschlossen. Der CPE beschreibt den mikroskopisch sichtbaren toxischen Effekt, den die Vermehrung der Viren in den Zellen auslöst. Die Adenoviren zerstören die Wirtszelle nach erfolgreicher Virusproduktion, die Zellen runden sich ab, lösen sich von der Zellkulturschale und die Membranen werden durchlässig. Bevorzugt sollten die Zellen abgeerntet werden, bevor die Viren freigesetzt werden. Nach Abzentrifugieren der Zelltrümmer können die im Überstand befindlichen Viren über einen Cäsiumchlorid-Gradienten aufgereinigt und anschließend dialysiert werden.

### [Ausführungsbeispiele]

### [Beispiel 1]

Menschliches Zellmaterial aus Knochenmark wurde durch Ausspülen mit Hilfe einer Kanüle gewonnen. Dabei wurde 5 ml sterile gepufferte physiologische Kochsalzlösung in das Knochenmark injiziert und die Zellen mit dieser Flüssigkeit in die Spritze gezogen. Die Zellen wurden anschließend bei 1200 rpm (Megafuge 1.0R, Kendro) für 10 min bei Raumtempertur pelletiert. Das Pellet wurde in 5 ml of 5 mM EDTA aufgenommen, wobei eine Lyse der Erythrozyten stattfindet. Nach exakt 30 sec. wurde ein gleiches Volumen von 1,8% NaCl zugegeben, um die Lyse zu stoppen (als Alternative zur hypotonen Lyse der Erythrozyten können die Stammzellen durch Dichtezentrifugation [Histopaque Gradient, Dichte 1.077 g/cm³, Sigma Chemicals,Co.] angereichert werden. Die Zellen wurden pelletiert [1200 rpm (Megafuge 1.0R, Kendro) für 10 min bei Raumtempertur] und in 5 ml IMDM Medium inklusive 20 % fetalem Kälberserum aufgenommen. Zur Vermeidung von Kontaminationen wurden folgende Antibiotika / Antimycotika zugegeben: Penicillin 100 IU/ml, Streptomycin 100 µg/ml, Gentamycin 50 µg/ml and Amphotericin B 1 µg/ml. Medium und Antibiotika werden im Folgenden als Vollmedium bezeichnet.

Die Zellen wurden gezählt und in einer Dichte von 0,2x10⁶ Zellen/cm² in Zellkultur-Petrischalen oder Zellkulturflaschen mit Vollmedium (s.o.) ausgesät. Es kann sinnvoll sein, die adhärenten adulten Stammzellen von den nicht adhärent wachsenden Zellen durch mehrmaliges Ausplattieren auf Petrischalen für mindestens 1 h und Entfernen der Suspensionszellen zu trennen. Die Kulturen wurden bei 37°C und 5% CO₂ inkubiert. Nach drei Tagen wurde die Hälfte des Mediums und damit die Hälfte der nicht-adhärenten Zellen entfernt und durch 37°C warmes Vollmedium ersetzt. Dieser Vorgang wurde nach weiteren 5-10 Tagen je nach Wachstum der Zellen wiederholt. Nach ca. 14 Tagen waren die Zellen konfluent, wurden mit Hilfe von Trypsin von der Kulturschale gelöst (Inkubation in 0,025% Trypsin/0,1 mM EDTA für 5 min) und zu 1x10⁴ cells/cm² in Zellkulturflaschen ausgesät. Nach 2-3 Passagen konnten die Zellen für die Experimente eingesetzt werden.

### [Beispiel 2]

Zur Markierung mesenchymaler Zellen, welche in die kardiogene Linie differenzieren, wurde ein System auf der Basis von Adenoviren angewendet, welches auf der Verwendung des MLC2v-Promotors beruht. Der Promotor kontrollierte die Expression eines Reportergens (GFL) bestehend aus dem ,green fluorescent protein' (GFP) und dem Gen für das Luziferase-Enzym, so dass Zellen, welche in die kardiogene Richtung differenzieren, anhand der grünen Färbung bzw. der Luziferase-Aktivität erkannt werden konnten.
Die GFL exprimierenden Zellen können anschließend mittels 'Fluorescence-activated cell sorting' (FACS) entweder über die Eigenfluoreszenz des GFP-Proteins oder nach Bindung Luziferase-spezifischer Antikörper, welche direkt mit Fluoreszenz-Molekülen konjugiert bzw. über einen Fluoreszenz-markierten Zweitantikörper detektierbar sind, isoliert werden.

Die Adenoviren mit GFL unter der Kontrolle des MLC2v-Promotors wurden nach der Methode von He et al. (1998, Proc Natl Acad Sci USA, 95:2509-14) generiert, wobei die homologe Rekombination zwischen einem die MLC2v-GFL-Sequenzen enthaltenden Plasmids und der Virus-DNA (ebenfalls in Form eines Plasmids) in Bakterien erfolgt. Als Ausgangskonstrukte für die Generierung rekombinanter Adenoviren, welche GFL unter der Kontrolle des MLC2v-Promotors exprimieren, wurden folgende Plasmide verwendet:
a) das Plasmid pCKC2R1, welches die Sequenzen des MLC2v-Promotors enthält,
b) das Plasmid pGFL mit dem aus dem ,green fluorescence protein' (GFP) und dem Luziferase-Enzym bestehenden Reportergen GFL,
c) der adenovirale Transfervektor pShuttle,
d) der den Großteil der adenoviralen Sequenzen enthaltende Vektor pAdEasy.

In einem ersten Schritt im Zuge der Adenovirus-Generierung wurde zunächst ein den MLC2v-Promotor enthaltendes Fragment aus dem Konstrukt pCKC2R1 isoliert. Dafür wurde pCKC2R1 mit dem Restriktionsenzym EcoRI linearisiert, die Enden mit Hilfe von T4-DNA-Polymerase und Nukleotiden aufgefüllt und mit KpnI nachgespalten. Dieses Fragment wurde vor die GFL-Sequenz des Plasmids pGFL inseriert. Dafür wurde pGFL mit MluI gespalten, mit Hilfe von T4-DNA-Polymerase und Nukleotiden an den Enden aufgefüllt und mit KpnI nachgespalten. Danach wurde das oben beschriebene, den MLC2v-Promotor enthaltende Fragment einligiert, womit das Konstrukt pMLC2v-GFL entstand.

Zur Herstellung des adenoviralen Transfervektors pAd-Shuttle-MLC2v-GFL wurde das Konstrukt pMLC2v-GFL mit NotI und SalI gespalten, das den MLC2v-Promotor mit angrenzenden GFL-Sequenzen enthaltende Fragment isoliert und in den ebenfalls mit NotI und SalI geschnittenen adenoviralen Transfervektor pShuttle einkloniert.

Zur homologen Rekombination wurden sowohl der adenovirale Vektor pAdEasy als auch der Transfervektor pAd-Shuttle-MLC2v-GFL mit PmeI linearisiert und beide Vektoren in BJ 5183 Bakterien über Co-Transformation eingebracht. Unter Co-Transformation versteht man das gleichzeitige Einbringen zweier oder mehrerer verschiedener Plasmide. Diejenigen Bakterien, welche über homologe Rekombination entstandene Adenovirusplasmide enthielten, wurden anschließend über die durch den Transfervektor eingebrachte Kanamycinresistenz selektioniert. Nach Isolierung und Analyse wurde die Adenovirusplasmid-DNA in Bakterien des Stamms HB101 amplifiziert.

Zur Generierung der rekombinanten Adenoviren wurden die Adenovirusplamide mit PacI linearisiert und über Ca-Phosphat-Präzipitation in die Helferzellinie 10SW transfiziert. Nach Lyse der Zellen wurden die ins Medium entlassenen Viren zur weiteren Infektion von 10SW Zellen verwendet. Dadurch wurde ein Virusstock mit hohem Titer erzeugt, der zur Infektion weiterer Zellen diente.

Zur Gewinnung gereinigter Viruspartikel wurde der durch die Viren hervorgerufene cytopathischen Effekts (CPE) abgewartet.

Nach Auftreten des CPE wurden die Zellen bei 500 g abzentrifugiert, in PBS (phosphate buffered saline) aufgenommen, und mit einem Glashomogenisator aufgeschlossen. Die nun einem geringen Volumen, z.B. 8 ml PBS, befindlichen Zellen wurden mit Cäsiumchlorid versetzt, so dass eine Dichte von 1.35 g/ml entsteht (bei 8 ml Viruslösung werden 4.4 g Cäsiumchlorid zugegeben). Diese Lösung wurde im SW41 Rotor bei 32.000 rpm für 18-24 Stunden bei 4°C zentrifugiert. Dabei bilden die Viren eine Bande im Gradienten. Die Zentrifugenröhrchen wurden unten mit einer Kanüle angestochen, es wurden 0,5 ml Fraktionen aufgenommen. Diese wurden auf ausplattierten 10SW Zellen im Plaque Assay getestet. Diejenigen Fraktionen, bei der die meisten Plaques gebildet wurden, wurden zusammengeführt und dann gegen ein 100faches Volumen von 10 mM Tris HCl, pH 8, bei 4°C dialysiert. Zum Einfrieren der Viren wurden zu 1 Volumen Virussuspension 1 Volumen doppelt konzentrierter Lagerungspuffer (10mM Tris HCl, pH 8, 100 mM NaCl, 0.1 % BSA, 20% Glycerol) gegeben. Die Lagerung erfolgte bei -80°C.

Zum Spezifitäts-Nachweis der Ad-MLC2v-GFL Adenoviren wurden einerseits Kardiomyozyten und andererseits nicht-kardiogene Zellen (Zellinien oder primäre Zellen wie beispielsweise mesenchymale Stammzellen) mit den Viren infiziert und 24h nach Infektion vergleichend die Expression des GFL-Proteins bestimmt. Zur Normalisierung wurden die Zellen parallel mit Adenoviren infiziert, welche das LacZ-Gen unter Kontrolle des RSV-Promotors besitzen, und die Infektionsraten über die Aktivität der β-Galaktosidase bestimmt.

Nachdem die Spezifität des MLC-2v-Promotors über die GFL-Expression in Herzmuskelzellen gezeigt wurde, wurden die MLC-2v-GFL enthaltenden Viren zur Bestimmung der Differenzierung mesenchymaler Stammzellen eingesetzt. Dazu wurden mesenchymale Stammzellen 7 Tage lang mit unterschiedlichen Konzentrationen 5'-Azacytidin (2,5 µM, 15 µM und 40 µM) behandelt, anschließend mit den Ad-MLC2v-GFL-Viren infiziert und 2 Tage nach Infektion aufgearbeitet. Die Bestimmung der MLC2v-Promotor-Aktivität erfolgte über Messen der Luziferase-Aktivität. Als Kontrolle dienten nicht mit 5'-Azacytidin behandelte MSC sowie neonatale Kardiomyozyten, welche ebenfalls mit den Ad-MLC2v-GFL-Viren infiziert wurden. Wie in Figure 1 dargestellt, nahm die Aktivität des MLC2v-Promotors im Gegensatz zu den unbehandelten Zellen mit steigender 5'-Azacytidin-Konzentration zu, was zeigt, daß ein Teil der Zellen nach Behandlung mit 5'-Azacytidin den Kardiomyozyten-spezifischen MLC2v-Promotor aktivierten und damit den Weg in die kardiomyogene Differenzierung eingeschlagen haben.

### [Beispiel 3]

Zur transienten Selektion kardiomyogener Stammzellen wurden Adenoviren verwendet, welche das Gen der Hygromycin-Phosphotransferase (hph) unter Kontrolle des MLC2v-Promotors enthalten. Die Hygromycin-Phosphotransferase vermittelt Resistenz gegenüber Hygromycin. Mit diesem Konstrukt können mesenchymale Stammzellen, welche den Weg in die kardiale Differenzierung einschlagen, über eine zeitlich begrenzte Gabe von Hygromycin selektioniert werden können.

Die Adenoviren mit dem Hygromycin-Phosphotransferase-Gen unter Kontrolle des MLC2v-Promotors wurden wie oben für MLC2v-GFL beschrieben nach der Methode von He et al. (1998, Proc Natl Acad Sci USA, 95:2509-14) generiert. Als Ausgangskonstrukte für die Generierung der rekombinanten Adenoviren standen folgende Plasmide zur Verfügung:
a) das Plasmid pMLC2v-GFL (siehe oben), welches die Sequenzen des GFL-Reportergens unter Kontrolle des MLC2v-Prömotors enthält,
b) das Plasmid pCMV-Hygro Hygromycin-Phosphotransferase-Gen unter Kontrolle des Cytomegalievirus (CMV)-Promotors
c) der adenovirale Transfervektor pShuttle,
d) der den Großteil der adenoviralen Sequenzen enthaltende Vektor pAdEasy.

Zur Herstellung des Konstrukts pMLC2v-Hygro wurden die GFL-Sequenzen aus dem Plasmid pMLC2v-GFL über Spaltung mit BglII entfernt und im Anschluß daran die Enden des linearisierten Plasmids aufgefüllt (mit Hilfe von T4-DNA-Polymerase und Nukleotiden). Parallel dazu wurde das das Hygromycinphosphotransferase-Gen enthaltende DNA-Fragment aus dem Plasmid pCMV-Hygro über EcoRI- und KpnI-Spaltung isoliert, dessen Enden ebenfalls mit Nukleotiden aufgefüllt und hinter den MLC2v-Promotor des linearisierten pMLC2v-GFL-Plasmids einkloniert.

Die Klonierung des adenoviralen Transfervektors pAd-Shuttle-MLC2v-Hygro erfolgte analog der Klonierung von pAd-Shuttle-MLC2v-GFL (siehe oben). Dazu wurde aus dem Plasmid pMLC2v-Hygro über Spaltung mit NotI und SalI ein Fragment isoliert, das den MLC2v-Promotor mit dem Hygromycinphosphotransferase-Gen enthielt. Dieses Fragment wurde in den mit den gleichen Restriktionsenzymen geschnittenen adenoviralen Transfervektor pShuttle kloniert.

Die homologe Rekombination der beiden adenoviralen Vektoren pAd-Shuttle-MLC2v-GFL und pAdEasy erfolgt wie oben für pAd-MLC2v-GFL beschrieben in Bakterien des Stammes BJ 5138. Diejenigen Bakterien, welche über homologe Rekombination entstandene Adenovirusplasmide enthielten, wurden anschließend über die durch den Transfervektor eingebrachte Kanamycinresistenz selektioniert. Nach Isolierung und Analyse wurde die Adenovirusplasmid-DNA in Bakterien des Stamms HB101 amplifiziert (vermehrt).

Zur Generierung der rekombinanten Adenoviren wurden die Adenovirusplamide mit PacI linearisiert und über Ca-Phosphat-Präzipitation in die Helferzellinie 10SW transfiziert. Nach Lyse der Zellen wurden die ins Medium entlassenen Viren zur weiteren Infektion von 10SW Zellen verwendet. Dadurch wurde ein Virusstock mit hohem Titer erzeugt, der zur Infektion weiterer Zellen diente.

Die Aufreinigung der Ad-MLC2v-Hygro-Viren erfolgte wie oben für Ad-MLC2v-GFL beschrieben über Cäsiumchlorid-Gradient.

### [Beispiel 4]

Zur Selektion von Stammzellen, welche nach 5'-Azacytidin-Behandlung zu kardiogenen Zellen differenzieren, wurden 3 x 10⁴ mesenchymale adulte Knochenmarkstammzellen auf 9,62 cm² ausgesät. Einen Tag später wurde 5'-Azacytidin zugegeben (Endkonzentration 40 µM) und die Zellen wurden nach weiteren 7 Tagen Kultur mit 1 x 10⁸ Ad-MLC2v-Hygro-Viren infiziert. Zwei Tage nach Infektion wurde den Zellen für 3 Tage Hygromycin B in einer Konzentration von 50 U/ml zugesetzt. Als Kontrollen dienen (1) mesenchymale Stammzellen, welche mit 5'-Azacytidin behandelt, aber nicht mit Ad-MLC2v-Hygro-Viren infiziert werden, (2) MSC, welche mit Ad-MLC2v-Hygro-Viren infiziert, jedoch nicht mit 5'-Azacytidin behandelt werden und (3) MSC, welche weder mit 5'-Azacytidin behandelt noch mit Ad-MLC2v-Hygro-Viren infiziert werden.
Nach der 3-tägigen Hygromycin-Behandlung überleben etwa 30-50% derjenigen mesenchymalen Stammzellen, welche sowohl mit den Adenoviren infiziert als auch der Behandlung mit 5'-Azacytidin unterzogen wurden, während sämtliche Zellen der Kontrollansätze, d.h. Zellen, welche weder mit Ad-MLC2v-Hygro transfiziert noch mit 5'-Azacytidin behandelt oder aber nur transfiziert bzw. nur mit 5'-Azacytidin behandelt wurden, in Anwesenheit von Hygromycin nicht überlebten.

### [Beispiel 5]

Der Verlust des Selektionskonstruktes wurde wie folgt nachgewiesen: Mesenchymale adulte Knochenmarkstammzellen wurden zu 3 x 10⁴ Zellen auf 9,62 cm² ausgesät. Einen Tag später wurde 5'-Azacytidin zugegeben (Endkonzentration 40 µM) und die Zellen wurden nach weiteren 7 Tagen Kultur mit 1 x 10⁸ Ad-MLC2v-Hygro-Viren infiziert. Zwei Tage nach Infektion wurde den Zellen für 7 Tage Hygromycin B in einer Konzentration von 50 units/ml zugesetzt. Es wurde gefunden, dass die Zellen vollständig abstarben, während bei einer kürzeren Hygromycin-Gabe ein Teil der Zellen überlebte. Folglich war die Expression des Hygromycin-Resistenzgens und damit auch der Verbleib der adenoviralen DNA in den Zellen nur transient.

### [Beispiel 6]

Zum Nachweis von GATA-4 mit Immunfluoreszenz wurden Herzmuskelzellen aus neugeborenen Ratten und mesenchymale Knochenmarkstammzellen aus Ratten auf mikroskopische Deckgläser aufgesät und kultiviert. Für die Immunfärbung wurden die Zellen mit 2% Formaldehyd fixiert. Primärer Antikörper war ein anti-GATA-4 (goat polyclonal, Santa Cruz Biotechnology Inc., Verdünnung 1:100), sekundärer Antikörper war ein biotinylierter anti-goat Antikörper (Vector Laboratories, Vedünnung 1:300). Zum Nachweis wurde mit Fluorescein gekoppeltes Avidin verwendet und die Zellen mit DAPI gegengefärbt.
In 5 und 11 Tage lang kultivierten Herzmuskelzellen aus neugeborenen Ratten war starke Immunfärbung gegen GATA-4 zu sehen. In 5 Tage lang kultivierten mesenchymalen Knochenmarkstammzellen war dagegen keine Immunfärbung erkennbar.

### [Beispiel 7]

Zum Nachweis von GATA-4 über Western Blotting wurden Proteinextrakte von Organen und Zellkulturen mit RIPA Puffer (in PBS: 0.5 % Na-deoxycholat, 1 % NP-40, 0.1 % SDS, 1 mM DTT, 0.5 mM Pefabloc SC (Roche), 1x vollständiger EDTA-freier Proteaseinhibitor-Cocktail (Roche), 6.25 µl/ml PSC-Protector-Solution (Roche)) angefertigt. Die Proteinkonzentration wurde nach der Lowry-Methode gemessen. Für SDS-Polyacrylamid-Gelelektrophorese wurden 50 µg der jeweiligen Extrakte wurden auf ein zehnprozentiges SDS-Gel geladen. Nach Western blotting wurde die primäre Antikörper-Inkubation mit anti-GATA-4 (goat polyclonal, Santa Cruz Biotechnology Inc., Verdünnung 1:100) durchgeführt, als Sekundär-Antikörper wurde ein Kaninchen anti-goat IgG konjugiert mit Meerrettich Peroxidase (horse radish peroxidase, PIERCE, Verdünnung 1:10 000) verwendet. Die Entwicklung erfolgte über Chemolumineszenz (Amersham ECL).
Mit dem Immunblot konnte GATA-4 in kultivierten Herzmuskelzellen neugeborener Ratten nach 3 und nach 5 Passagen der Zellen nachgewiesen werden. Besonders stark war das Signal in Herzgewebe neugeborener Ratten, schwächer in Herzgewebe adulter Ratten. In adulten mesenchymalen Knochenmarkstammzellen der Ratte war mit dieser Methode dagegen kein GATA-4 nachweisbar.

### [Beispiel 8]

Zum Nachweis von Serca2a mit Immunfluoreszenz wurden Herzmuskelzellen aus neugeborenen Ratten und mesenchymale Knochenmarkstammzellen auf mikroskopische Deckgläser aufgesät und kultiviert. Für die Immunfärbung wurden die Zellen mit 2% Formaldehyd fixiert. Primärer Antikörper war ein anti-Serca2a (Santa Cruz, Verdünnung 1:100), sekundärer Antikörper war ein biotinylierter anti-goat Antikörper (Vector Laboratories, Vedünnung 1:300). Zum Nachweis wurde mit Fluorescein gekoppeltes Avidin verwendet und die Zellen mit DAPI gegengefärbt. Für Herzmuskelzellen neugeborener Ratten wurde in einem weiteren Versuch auch mit Rhodamin gekoppeltes Avidin verwendet.

In 8 Tage lang kultivierten Herzmuskelzellen aus neugeborenen Ratten war starke Immunfärbung gegen Serca2a zu sehen. In 8 Tage lang kultivierten mesenchymalen Knochenmarkstammzellen war dagegen lediglich schwache Immunfärbung als vermutlicher Artefakt durch die Signalamplifikation erkennbar.

### [Beispiel 9]

Zum Nachweis von anti-Cardiac Troponin I mit Immunfluoreszenz wurden Herzmuskelzellen aus neugeborenen Ratten und mesenchymale Knochenmarkstammzellen der Ratte auf mikroskopische Deckgläser aufgesät und kultiviert. Für die Immunfärbung wurden die Zellen mit 2% Formaldehyd fixiert. Primärer Antikörper war ein anti-Cardiac Troponin I (Maus monoklonal, HyTest Ltd., Finland, Verdünnung 1:300), sekundärer Antikörper war ein biotinylierter anti-Maus Antikörper (Vector Laboratories, Vedünnung 1:300). Zum Nachweis wurde mit Fluorescein gekoppeltes Avidin verwendet und die Zellen mit DAPI gegengefärbt. Für die Herzmuskelzellen neugeborener Ratten wurde in einem weiteren Versuch auch mit Rhodamin gekoppeltes Avidin verwendet.

In 5 und 8 Tage lang kultivierten Herzmuskelzellen aus neugeborenen Ratten war starke Immunfärbung gegen Cardiac Troponin I zu sehen. In 8 Tage und 5 Tage lang kultivierten mesenchymalen Knochenmarkstammzellen war dagegen keine Immunfärbung erkennbar.

### [Beispiel 10]

Zum Nachweis von anti-Cardiac Troponin I über Western Blotting wurden Proteinextrakte von Organen und Zellkulturen mit RIPA Puffer (in PBS: 0.5.% Na-deoxycholat, 1 % NP-40, 0.1 % SDS, 1 mM DTT, 0.5 mM Pefabloc SC (Roche), 1x vollständiger EDTA-freier Proteaseinhibitor-Cocktail (Roche), 6.25 µl/ml PSC-Protector-Solution (Roche)) angefertigt. Die Proteinkonzentration wurde nach der Lowry-Methode gemessen. Für SDS-Polyacrylamid-Gelelektrophorese wurden 50 µg der jeweiligen Extrakte auf ein zehnprozentiges SDS-Gel geladen. Nach Western blotting wurde die primäre Antikörper-Inkubation mit anti-Cardiac Troponin I (Maus monoklonal, HyTest Ltd., Finland, Verdünnung 1:600) durchgeführt, als Sekundär-Antikörper wurde ein Ziegen anti-mouse IgG konjugiert mit Meerrettich Peroxidase (Santa Cruz Biotechnology Inc., Verdünnung 1:5000) verwendet. Die Entwicklung erfolgte über Chemolumineszenz (Amersham ECL).

Mit dem Immunblot konnte Cardiac Troponin I von der erwarteten Größe von 29 kDa sowohl in Herzgewebe neugeborener als auch adulter Ratten in etwa gleicher Stärke nachgewiesen werden. In adulten mesenchymalen Knochenmarkstammzellen der Ratte war mit dieser Methode dagegen kein Cardiac Troponin I von 29 kDa nachweisbar. Lediglich zwei schwache Banden für deutlich größere Proteine waren erkennbar, die als unspezifische Bindung des Antikörpers interpretiert werden. Cardiac Troponin I war nicht in Gewebeproben aus Leber, Lunge oder Skelettmuskel nachweisbar, was die Spezifität der Expression für Herzgewebe belegt.
In menschlichen Fibroblasten und menschlichen mesenchymalen Knochenmarkstammzellen konnte kein Cardiac Troponin I nachgewiesen werden, wobei der Antikörper durchaus in der Lage ist, menschliches Cardiac Troponin I zu binden, wie mittels Nachweis des menschlichen ITC Komplex gezeigt werden konnte.
Interessanterweise konnte in kommerziell erhältlichen humanen Herzmuskelzellen (Cellsystems Biotechnologie Vertriebs GmbH) kein Cardiac Troponin I nachgewiesen werden, diese kultivierten Zellen unterscheiden sich also von endogenen Herzmuskelzellen.

### [Beispiel 11]

Zum Nachweis von sarcomerem α-Aktin mit Immunfluoreszenz wurden Herzmuskelzellen aus neugeborenen Ratten und mesenchymale Knochenmarkstammzellen der Ratte auf mikroskopische Deckgläser aufgesät und kultiviert. Für die Immunfärbung wurden die Zellen mit 2% Formaldehyd fixiert. Primärer Antikörper war ein gegen sarcomeres α-Aktin gerichteter Antikörper (Maus monoklonal, Sigma, Verdünnung 1:500), sekundärer Antikörper war ein biotinylierter anti-Maus Antikörper (Vector Laboratories, Vedünnung 1:300). Zum Nachweis wurde mit Fluorescein gekoppeltes Avidin verwendet und die Zellen mit DAPI gegengefärbt. Für die Herzmuskelzellen neugeborener Ratten wurde in einem weiteren Versuch auch mit Rhodamin gekoppeltes Avidin verwendet.

In 5 und 8 Tage lang kultivierten Herzmuskelzellen aus neugeborenen Ratten war starke Immunfärbung gegen sarcomeres α-Aktin zu sehen. In 8 Tage lang kultivierten mesenchymalen Knochenmarkstammzellen war dagegen lediglich schwache Immunfärbung als vermutlicher Artefakt durch die Signalamplifikation erkennbar.

### [Beispiel 12]

Zum Nachweis von α-Myosin Heavy Chain mit Immunfluoreszenz wurden Herzmuskelzellen aus neugeborenen Ratten und mesenchymale Knochenmarkstammzellen der Ratte auf mikroskopische Deckgläser aufgesät und kultiviert. Für die Immunfärbung wurden die Zellen mit 2% Formaldehyd fixiert. Der primäre Antikörper war gegen α-Myosin Heavy Chain gerichtet (Maus monoklonal, Abcam Ltd., Großbritannien, Verdünnung 1:250), sekundärer Antikörper war ein biotinylierter anti-Maus Antikörper (Vector Laboratories, Verdünnung 1:300). Zum Nachweis wurde mit Fluorescein oder Rhodamin gekoppeltes Avidin verwendet und die Zellen mit DAPI gegengefärbt.

In 8 Tage lang kultivierten Herzmuskelzellen aus neugeborenen Ratten war starke Immunfärbung gegen α-Myosin Heavy Chain zu sehen. In 8 Tage lang kultivierten mesenchymalen Knochenmarkstammzellen war dagegen keine Immunfärbung erkennbar.

### [Kurze Beschreibung der Abbildungen]

### Fig. 1:

Humane mesenchymale Knochenmarkstammzellen (hMSC) bzw. humane fetale Herzmuskelzellen (hHMC) wurden in einer Dichte von 3x10³ Zellen / cm² ausgesät. Zwei Tage später wurden die Zellen mit 5'-Azacytidin in den angegebenen Konzentrationen von 0 bis 40µM behandelt. 7 Tage später wurden die Zellen mit den rekombinanten Adenoviren Ad-MLC2-GFL und Ad-RSV-LacZ co-transformiert, jeweils mit 50 pfu/Zelle. Ad-MLC-2-GFL exprimiert ein Fusionsprotein von GFP und Luciferase unter Kontrolle des MLC-2v-Promotors. Ad-RSV-LacZ exprimiert β-Galaktosidase (LacZ) unter Kontrolle des in allen Zellen ubiquitär aktiven Rous Sarcoma Virus (RSV)-Promotors. Zwei Tage nach Infektion wurden die Zellen geerntet und die Luciferase-Aktivität (RLU, relative light units) bestimmt. Die Luciferase-Werte wurden auf die Protein-Konzentration normalisiert und auf diese Weise für unterschiedliche Zellzahlen in den Proben korrigiert. Da eine gleichzeitige Infektion mit Ad-RSV-LacZ durchgeführt worden war, konnten die Luciferase-Werte in Bezug auf unterschiedliche Infektionseffizienzen durch quantitative Messung der β-Galaktosidase (LacZ) Aktivität (Umsatz . von ONPG, o-nitrophenyl-β-d-galactopyranosid) korrigiert werden.

Gezeigt ist die absolute Luciferase-Aktivität, normalisiert auf Protein-Konzentration und Infektionseffizienz. Gezeigt sind Mittelwerte von jeweils drei Messungen. Die Fehlerbalken zeigen die Standard-Abweichungen. Der MLC-2v-Promotor induziert die Expression von Luciferase (GFL) bei Zugabe bereits geringer Konzentrationen von 5'-Azacytidin.

### Fig. 2:

Plasmidkarte des Vektors pMLC2v-GFL

### Fig. 3:

Plasmidkarte des Vektors pMLC2v-Hygro

### Fig. 4:

Plasmidkarte des Vektors pShuttle

### Fig. 5:

Plasmidkarte des Vektors pAd-Shuttle-MLC2v-GFL

### Fig. 6:

Plasmidkarte des Vektors pAd-Shuttle-MLC2v-Hygro

### Erläuterung der Abkürzungen in Fig. 1 bis Fig. 6:

Bezeichungen außerhalb der Kreise bezeichnen die Schnittstellen der mit ihren international üblichen Abkürzungen bezeichneten Restriktionsenzyme.
- Ad5-L: Homologieregion zum linken Arm des Ad5 Adenovirus (Ad5 left arm homology region)
- Ad5-R: Homologieregion zum rechten Arm des Ad5 Adenovirus (Ad5 right arm homology region)
- AmpR: Ampicillin-Resistenzgen
- colE1: ColE1 origin of replication
- GFL: Sequenz kodierend für ein Fusionsprotein aus EGFP und Luciferase
- Enc: Encapsidation signal
- f1 ori: f1 origin of replication
- HygroR: Hygromycin-Resistenzgen
- L-ITR: linkes inverted terminal repeat
- R-ITR: rechtes inverted terminal repeat
- KanR: Kanamycin-Resistenzgen
- MLC2v-P: MLC-2v Promotor
- pBR322 ori: pBR322 origin of replication
- polyA: synthetisches Signal für Polyadenylierung
- SV401pA: SV40 late polyadenylation signal (Signalsequenz für Polyadenylierung abgeleitet von den Capsidprotein-Genen von SV40)
- Tk pA: Signalsequenz für Polyadenylierung des Herpes Simplex Virus Thymidinkinase-Gens

## Patentansprüche

1. Verfahren zur in vitro Selektion von kardiomyogenen Zellen oder Herzmuskelzellen aus einer Mischung mit anderen Zelltypen, enthaltend die Abfolge der folgenden Schritte:
a) Einbringen mindestens eines Selektionskonstruktes, das auf einem nicht in das zelluläre Genom integrierenden Vektor enthalten ist, in die zu selektierenden Zellen, wobei auf dem Selektionskonstrukt mindestens ein Selektionsmarker kodiert ist, der spezifisch in kardiomyogenen Zellen oder Herzmuskelzellen exprimiert wird,
b) Kultivierung der Zellen unter Zugabe mindestens eines cytotoxischen oder cytostatischen Selektionsmittels bis zum Erhalt einer reinen Zellpopulation kardiomyogener Zellen oder Herzmuskelzellen.

2. Verfahren gemäß Anspruch 1, **dadurch gekennzeichnet, dass** der Vektor viraler Herkunft ist, bevorzugt abgeleitet aus der Gruppe bestehend aus
a) Epstein-Barr-Virus (EBV),
b) Simian Virus 40 (SV40),
c) BK-Virus,
d) JC-Virus,
e) Adenovirus.

3. Verfahren gemäß Anspruch 1 oder 2, **dadurch gekennzeichnet, dass** das Gen für den Selektionsmarker unter Kontrolle einer Expressionssequenz steht, die einen Promotor ausgewählt aus der Gruppe bestehend aus
a) MLC-2v Promotor
b) α-Myosin-Heavy-Chain Promotor
c) GATA-4 Promotor
d) Cardiac Troponin I Promotor
e) Serca 2a Promotor
f) kardialer sarcomerer α-Actin Promotor
g) Nkx 2.5 Promotor
h) Myogenin Promotor
umfasst.

4. Verfahren gemäß einem der Ansprüche 1 bis 3, **dadurch gekennzeichnet, dass** das Selektionsmittel ein gegen tierische oder menschliche Zellen wirksames Antibiotikum enthält.

5. Verfahren gemäß einem der Ansprüche 1 bis 4, **dadurch gekennzeichnet, dass** das
Selektionsmarkergen ausgewählt ist aus der Gruppe von Genen kodierend für Neomycin-Phosphotransferase II, Neomycin-Phosphotransferase I, Hygromycin-Phosphotransferase, Blasticidin S Deaminase von Aspergillus terreus, Puromycin N-acetyl-Transferase, Sh ble von Streptoalloteichus Hindustanus,
und dass die Selektionsmittel ausgewählt sind aus der Gruppe bestehend aus Neomycin, Kanamycin, Paromomycin, Geneticin (G418), Hygromycin, Blasticidin, Puromycin, Zeocin/Phleomycin.

6. Verfahren gemäß einem der Ansprüche 1 bis 5, **dadurch gekennzeichnet, dass** die Kultivierung unter Zugabe des Selektionsmittels in Schritt b) für 2 bis 21 Tage, bevorzugt 3 bis 10 Tage und besonders bevorzugt für 3 bis 5 Tage erfolgt.

7. Verfahren gemäß einem der Ansprüche 1 bis 6, **dadurch gekennzeichnet, dass** die kardiomyogenen Zellen oder Herzmuskelzellen aus adulten Stammzellen, bevorzugt aus mesenchymalen adulten Stammzellen, differenziert wurden.

8. Verfahren gemäß Anspruch 7, **dadurch gekennzeichnet, dass** das nicht integrierende Selektionskonstrukt in die adulten Stammzellen eingebracht wurde.

9. Verfahren gemäß einem der Ansprüche 7 bis 8, **dadurch gekennzeichnet, dass** die Differenzierung durch Kokultivierung mit Herzmuskelzellen ausgelöst wird oder durch Mittel enthaltend mindestens eine der Substanzen ausgewählt aus der Gruppe bestehend aus
a) DNA-demethylierende Substanzen, bevorzugt 5'-Azacytidin
b) Inhibitoren der Histondeazetylierung, bevorzugt Trichostatin A
c) Amphotericin B
d) Dexamethason
e) Retinsäure
f) Proteine, die eine Differenzierung zu kardiomyogenen oder Herzmuskelzellen auslösen können, bevorzugt aus der Gruppe bestehend aus Activin, TGF-beta, BMP-2, BMP-4, FGF-4, MyoD, GATA-4 und Nkx 2.5

10. Verfahren gemäß einem der Ansprüche 1 bis 9, **dadurch gekennzeichnet, dass** die kardiomyogenen Zellen oder Herzmuskelzellen gewebetypisiert wurden oder von Zellen eines gewebetypisierten Spenders abstammen.

11. Verfahren gemäß einem der Ansprüche 1 bis 10, wobei die Zellen während des Verfahrens vermehrt werden.

12. Verfahren zur Herstellung reiner Kulturen kardiomyogener Zellen oder Herzmuskelzellen gemäß dem Verfahren nach einem der Ansprüche 1 bis 11, zusätzlich enthaltend die Schritte
c) Entfernung des Selektionsmittels
d) Weiterkultivierung der Zellen bis zum Verlust des Selektionskonstruktes und gegebenenfalls des Selektionsmarkers.

13. Verfahren gemäß Anspruch 12, **dadurch gekennzeichnet, dass** während der Weiterkultivierung die Zellen vermehrt werden.

14. Verfahren gemäß Anspruch 12 oder 13, **dadurch gekennzeichnet, dass** die Weiterkultivierung in Schritt d) für 1 bis 12 Wochen, bevorzugt 4 bis 8 Wochen erfolgt.

15. Verfahren gemäß einem der Ansprüche 12 bis 14, **dadurch gekennzeichnet, dass** während der Weiterkultivierung in Schritt d) mindestens zwei Zellpassagen durchgeführt werden.

16. Reine Kultur kardiomyogener Zellen oder Herzmuskelzellen erhältlich nach dem Verfahren nach einem der Ansprüche 12 bis 15.

17. Verwendung kardiomyogener Zellen oder Herzmuskelzellen erhältlich aus einer reinen Kultur gemäß Anspruch 16 zur Herstellung eines Arzneimittels.

18. Arzneimittel enthaltend kardiomyogene Zellen oder Herzmuskelzellen erhältlich aus einer reinen Kultur gemäß Anspruch 16, sowie gegebenenfalls einen geeigneten pharmazeutischen Träger.

19. Zellkultur-Bank enthaltend reine Kulturen kardiomyogener Zellen oder Herzmuskelzellen gemäß Anspruch 16, wobei die Kulturen gewebetypisiert sind.

20. Reine Zellpopulation kardiomyogener Zellen oder Herzmuskelzellen, erhältlich nach einem der Ansprüche 1-11.

21. Verfahren zur Herstellung einer Gewebekultur enthaltend kardiomyogene Zellen oder Herzmuskelzellen, enthaltend die folgenden Schritte
a) Herstellung einer reinen Kultur kardiomyogener Zellen oder Herzmuskelzellen gemäß einem der Ansprüche 12 bis 15,
b) Aufbringen von Zellen aus der Kultur aus Schritt a) auf eine Gewebekultur Matrix,
c) Weiterkultivierung bis zur Entwicklung eines geschlossenen Zellverbandes auf der Gewebekultur-Matrix

22. Gewebekultur erhältlich gemäß dem Verfahren nach Anspruch 21.

23. Nicht in das zelluläre Genom integrierender Vektor viraler Herkunft, **dadurch gekennzeichnet, dass** er mindestens eine spezifisch in Herzmuskelzellen aktive Expressionssequenz enthält, die die Expresssion eines Selektionsmarkergens kontrolliert.
